(19) Europäisches Patentamt
European Patent Office
Office européen des brevets

(11) EP 2 182 357 A1

(12) **EUROPEAN PATENT APPLICATION**

(43) Date of publication:
**05.05.2010 Bulletin 2010/18**

(51) Int Cl.:
***G01N 33/543*** *(2006.01)*

(21) Application number: **09012973.5**

(22) Date of filing: **14.10.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **17.10.2008 EP 08018195**
**21.04.2009 EP 09005565**

(71) Applicants:
- **Roche Diagnostics GmbH**
**68305 Mannheim (DE)**
Designated Contracting States:
**DE**
- **F.HOFFMANN-LA ROCHE AG**
**4070 Basel (CH)**
Designated Contracting States:
**AT BE BG CH CY CZ DK EE ES FI FR GB GR HR HU IE IS IT LI LT LU LV MC MK MT NL NO PL PT RO SE SI SK SM TR**

(72) Inventors:
- **Fila, Claudia**
**81371 München (DE)**
- **Fritton, Hans-Peter**
**69509 Moerlenbach (DE)**
- **Kuenemund, Volker**
**69198 Schriesheim (DE)**
- **Watzele, Manfred**
**82362 Weilheim (DE)**

(54) **Cell monitoring and molecular analysis**

(57) The present invention provides a method for the real time analysis of cell cultures and their molecular content. More precisely, the present invention provides a method to monitor the cellular reaction of cells to certain stimuli in real time in order to figure out a reasonable point of time to perform an analysis of the molecular content of said cells.

**Fig. 1**

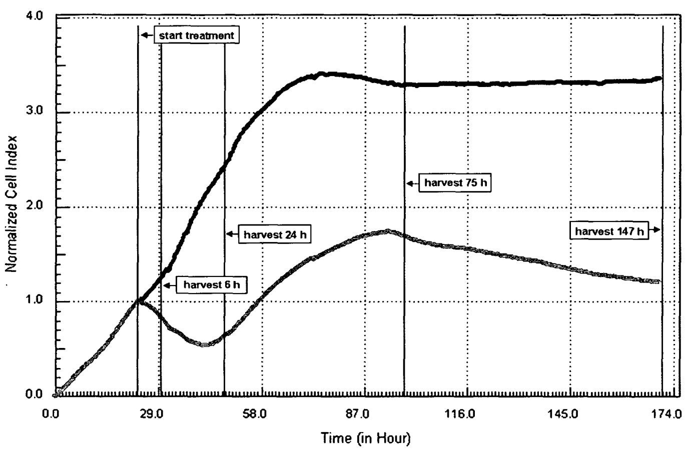


EP 2 182 357 A1

SEQUENCE LISTING

<110> Roche Diagnostics GmbH
F. Hoffmann-La Roche AG

<120> Cell monitoring and molecular analysis

<130> 25427 EP2

<150> 08018195
<151> 2008-10-17

<150> 09005565
<151> 2009-04-21

<160> 46

<170> PatentIn version 3.5

<210> 1
<211> 19
<212> DNA
<213> Artificial

<220>
<223> forward primer HDAC3

<400> 1
tgcattgtgc tccagtgtg 19

<210> 2
<211> 22
<212> DNA
<213> Artificial

<220>
<223> reverse primer HDAC3

<400> 2
ccttacgcaa cttatacagt tc 22

<210> 3
<211> 20
<212> DNA
<213> Artificial

<220>
<223> forward primer GNA11

<400> 3
gcatccagga atgctacgac 20

<210> 4
<211> 20
<212> DNA
<213> Artificial

<220>
<223> reverse primer GNA11

<400> 4
gatggactgg ctgcaactgg 20

<210> 5
<211> 20
<212> DNA
<213> Artificial

<220>
<223> forward primer ISG15

<400> 5
gcgaactcat ctttgccagt 20

<210> 6
<211> 20
<212> DNA
<213> Artificial

<220>
<223> reverse primer ISG15

<400> 6
ttgcttaagg tccacaggga 20

<210> 7
<211> 19
<212> DNA
<213> Artificial

<220>
<223> forward primer IFITM1

<400> 7
cctttgcact ccactgtgc 19

<210> 8
<211> 19
<212> DNA
<213> Artificial

<220>
<223> reverse primer IFITM1

<400> 8
gaaccaggac ggggatcta 19

<210> 9
<211> 24
<212> DNA
<213> Artificial

<220>
<223> forward primer BNIP3

<400> 9
gaatttctga aagttttcct tcca 24

<210> 10
<211> 20
<212> DNA
<213> Artificial

<220>
<223> reverse primer BNIP3

<400> 10
ataaccttcc gcagactgtt 20

<210> 11
<211> 20
<212> DNA
<213> Artificial

<220>
<223> forward primer SLUG

<400> 11
tggttgcttc aaggacacat 20

<210> 12
<211> 19
<212> DNA
<213> Artificial

<220>
<223> reverse primer SLUG

<400> 12
aagaacggga gtgacgttg 19

<210> 13
<211> 20
<212> DNA

<213> Artificial

<220>
<223> forward primer FOS

<400> 13
ctaccactca cccgcagact 20

<210> 14
<211> 19
<212> DNA
<213> Artificial

<220>
<223> reverse primer FOS

<400> 14
tcctgaagac gtgcctgga 19

<210> 15
<211> 18
<212> DNA
<213> Artificial

<220>
<223> forward primer ARF-1

<400> 15
ttcgccaaca agcaggac 18

<210> 16
<211> 20
<212> DNA
<213> Artificial

<220>
<223> reverse primer ARF-1

<400> 16
agtgatgcgg tgtccttgac 20

<210> 17
<211> 21
<212> DNA
<213> Artificial

<220>
<223> forward primer CDKN1A

<400> 17
cgaagtcagt tccttgtgga g 21

<210> 18
<211> 19
<212> DNA
<213> Artificial

<220>
<223> reverse primer CDKN1A

<400> 18
tacaggcagt cttgggtac 19

<210> 19
<211> 20
<212> DNA
<213> Artificial

<220>
<223> forward primer PIG8

<400> 19
tggtgaagag atggctgaca 20

<210> 20
<211> 23
<212> DNA
<213> Artificial

<220>
<223> reverse primer PIG8

<400> 20
agaccccata aacatggtag agt 23

<210> 21
<211> 24
<212> DNA
<213> Artificial

<220>
<223> forward primer CDC2

<400> 21
catggatctg aagaaatact tgga 24

<210> 22
<211> 20
<212> DNA
<213> Artificial

<220>
<223> reverse primer CDC2

<400> 22
ggtttaggat gtcccctaac 20

<210> 23
<211> 18
<212> DNA
<213> Artificial

<220>
<223> forward primer PLAB

<400> 23
ccggatactc acgccaga 18

<210> 24
<211> 20
<212> DNA
<213> Artificial

<220>
<223> reverse primer PLAB

<400> 24
tggacgtgga cgcatagaga 20

<210> 25
<211> 22
<212> DNA
<213> Artificial

<220>
<223> forward primer TOP2A

<400> 25
ttgtggaaag aagacttggc ta 22

<210> 26
<211> 22
<212> DNA
<213> Artificial

<220>
<223> reverse primer TOP2A

<400> 26
ggttcctttt tgttctactt gt 22

<210> 27
<211> 25
<212> DNA

<213> Artificial

<220>
<223> forward primer MADH2 / SMAD2

<400> 27
ctaaatgtgt taccatacca agcag 25

<210> 28
<211> 20
<212> DNA
<213> Artificial

<220>
<223> reverse primer MADH2 / SMAD2

<400> 28
tagcttttcc taacggtgta 20

<210> 29
<211> 22
<212> DNA
<213> Artificial

<220>
<223> forward primer ATF2

<400> 29
tgaccgaaag gatcatgaac ta 22

<210> 30
<211> 22
<212> DNA
<213> Artificial

<220>
<223> reverse primer ATF2

<400> 30
acctttgaac tctttcctga cg 22

<210> 31
<211> 18
<212> DNA
<213> Artificial

<220>
<223> forward primer SPRY4

<400> 31
ccccggcttc aggattta 18

<210> 32
<211> 20
<212> DNA
<213> Artificial

<220>
<223> reverse primer SPRY4

<400> 32
gacataactc gccaaacgtc 20

<210> 33
<211> 21
<212> DNA
<213> Artificial

<220>
<223> forward primer LIPA

<400> 33
gctggaactt ctgtgcaaaa c 21

<210> 34
<211> 21
<212> DNA
<213> Artificial

<220>
<223> reverse primer LIPA

<400> 34
tcaaagttcg gaaactgacc c 21

<210> 35
<211> 21
<212> DNA
<213> Artificial

<220>
<223> forward primer IDI1

<400> 35
gctaggaatt cccttggaag a 21

<210> 36
<211> 21
<212> DNA
<213> Artificial

<220>
<223> reverse primer IDI1

<400> 36
cagactacca tagaccccac t 21

<210> 37
<211> 18
<212> DNA
<213> Artificial

<220>
<223> forward primer FDPS

<400> 37
gagtacccgc caacaagc 18

<210> 38
<211> 18
<212> DNA
<213> Artificial

<220>
<223> reverse primer FDPS

<400> 38
acagggcgac caactcta 18

<210> 39
<211> 19
<212> DNA
<213> Artificial

<220>
<223> forward primer IGFBP5

<400> 39
accgcgagca agtcaagat 19

<210> 40
<211> 18
<212> DNA
<213> Artificial

<220>
<223> reverse primer IGFBP5

<400> 40
actctaccgg ctcctctg 18

<210> 41
<211> 18
<212> DNA

<213> Artificial

<220>
<223> forward primer ß-Actin

<400> 41
attggcaatg agcggttc 18

<210> 42
<211> 17
<212> DNA
<213> Artificial

<220>
<223> reverse primer ß-Actin

<400> 42
ggatgccagg actccat 17

<210> 43
<211> 19
<212> DNA
<213> Artificial

<220>
<223> forward primer ß-Globin

<400> 43
tgcaggctgc ctatcagaa 19

<210> 44
<211> 22
<212> DNA
<213> Artificial

<220>
<223> reverse primer ß-Globin

<400> 44
gcgagcttag tgatacttgt gg 22

<210> 45
<211> 20
<212> DNA
<213> Artificial

<220>
<223> forward primer GAPDH

<400> 45
ctctgctcct cctgttcgac 20

```
<210>  46
<211>  20
<212>  DNA
<213>  Artificial

<220>
<223>  reverse primer GAPDH

<400>  46
acgaccaaat ccgttgactc                                                    20
```

## Description

## Background of the Invention

Changes in expression patterns of genes can be detected through genome-wide expression profiling using commercially available gene chip microarray technology (e.g. from Affymetrix, Illumina or NimbleGen). Measuring the relative amount of mRNA expressed under, ideally, two experimental conditions (non-treated versus compound-treated) at different time points upon compound administration creates a global picture of cellular changes in response to the compound. A modem low-throughput approach for measuring mRNA abundance is provided by the quantitative Real-time Polymerase chain reaction (q-RT-PCR), e.g. applying the LightCycler® Systems of Roche Diagnostics GmbH. It enables both, the detection and quantification (as absolute number of copies or relative amount when normalized by the copy number of house-keeping genes as relatively stably expressed internal reference gene) of a specific sequence in a DNA/cDNA (when reverse transcribed from mRNA) sample. q-RT-PCR is the gold standard for validating data generated from microarrays or for the quantification of specific and pre-defined transcript levels, whenever quantitative data, reproducibility and comparability between several projects are required. Thus, this method can be used either to repeat and validate data generated from microarray experiments or for hypothesis-driven large or small scale expression screens (e.g. specific panel of functionally related genes) based solely on q-RT-PCR as expression profiling technique. While high throughput DNA microarrays lack the quantitative accuracy of the q-RT-PCR, it takes about the same time to measure the gene expression of a few dozen genes via q-RT-PCR or to measure an entire genome using DNA microarrays. So it often makes sense to perform semi-quantitative DNA microarray analysis experiments to identify candidate genes and then perform a q-RT-PCR on some of the most interesting candidate genes to validate the microarray results. The ability to generate sensitive and specific gene expression profiles are fundamental, especially in the identification of drug targets and revealing the mechanisms of drug resistance.

However, global as well as large scale expression profiling using this kind of experimental set-up is time-consuming and expensive. Often it is difficult to determine and set the right time point for a gene expression analysis and multiple experiments have to be conducted at randomly chosen time points upon compound treatment. But financial constraints limit expression profiling experiments to a small number of measurements for a single gene at a given time point under identical conditions or to a small number of different conditions or to a small number of different time points upon altering a condition. Consequently, this reduces the statistical power of an experiment, making it impossible for the experiment to identify important subtle gene expression changes.

Usually, gene expression profiling on the RNA level is monitored on routine basis by a multi-step procedure. First, the respective cellular sample is removed from the culture vessel. In case of adherent cells harvesting may be supported by trypsination (treatment with a Trypsin-EDTA solution) in order to detach the adherent cells from the solid support. Secondly, the collected cells are pelleted and subjected to cell lysis. As a third step it is usually required to at least partially purify the total RNA or mRNA that is present in the sample (EP 0 389 063). Afterwards, a first strand cDNA synthesis step is performed with an RNA dependent DNA polymerase such as AMV or MMuLV Reverse Transcriptase (Roche Applied Science).

Subsequently, the amount of generated cDNA is quantified either by means of quantitative PCR (Sagner, G., and Goldstein, C., BIOCHEMICA No: 3 (2001) 15-17) or alternatively by means of amplification and subsequent hybridization onto a DNA microarray (Kawasaki, E.S., Ann. N.Y. Acad. Sci. 1020 (2004) 92-100). In case of PCR, a one step RT-PCR may be performed, **characterized in that** the first strand cDNA synthesis and subsequent amplification are catalyzed by the same Polymerase such as T.th Polymerase (Roche Applied Science Cat. No. 11 480 014).

In traditional real time RT-PCR or qRT-PCR, RNA is first isolated from cells with procedures that can lead to a loss of material. Using the CellsDirect cDNA Synthesis System (Invitrogen Cat No. 11737-030), the cells are lysed and the cDNA is generated from the lysate in a single tube with minimal handling and no sample loss. DNase I is added to eliminate genomic DNA prior to first-strand synthesis. After synthesis, the first-strand cDNA can be transferred directly to the qPCR reaction without intermediate organic extraction or ethanol precipitation. This kit has been optimized for small cell samples, ranging from 10,000 cells down to a single cell.

Within the field of cellular analysis based on living cells, real time monitoring prior to the analysis of the molecular content on nucleic acid or protein level is mainly performed using optical techniques. Many of these optical techniques are endpoint assays requiring a tedious labeling procedure and fixation of the cells. These fixation steps usually prevent a further downstream analysis. For screening applications (e.g. screening of different chemical stimuli for a certain cell type or screening of different cell types for a certain chemical stimuli), the amount of cellular information obtainable from optical techniques is limited and consequently, the time points after a certain cell stimulus for a certain downstream analysis is in general defined by empirical values and not by real time monitoring of the living cells.

This experimental strategy bares the risk that said downstream analysis is performed to early, namely before the expected reaction based on the stimulus takes place, or too late, namely after the expected reaction based on the stimulus already subsided. Moreover, this end-point strategy may miss certain intermediate reactions of the living cells.

The continuous monitoring of cellular features, such as adhesion, morphology, locomotion, growth and viability would allow the determination of the appropriate time point(s) by correlating drug-induced changes of cellular behavior of whatever quality and extent with preceding or concomitant changes in expression of genes potentially involved in the observed cellular effect. In this way it would also be possible to discriminate between very rapid and often short-lasting cellular effects that are usually based on changes in adhesion, locomotion and morphology from later and rather long-lasting effects due to changes in viability and/or growth that are primarily based on alterations in expression of genes involved in cell proliferation, apoptosis and metabolism.

A non-optical technique providing a much higher analytical content known to someone skilled in the art of cellular analysis is impedance measurement. Here, the cells are cultured on electrode arrays and the properties of the cells can be analyzed using electrical stimuli in real time. A commercial system for cell analysis based on impedance measurements is for example the xCELLigence® system of Roche Diagnostics GmbH.

The combination of real-time monitoring of cells with a technique that analyses the molecular content of cells offers the advantage that the information about the time point of cellular changes in response to the treatment with a certain compound and the appropriately timed co-application of the molecular analysis increases the efficiency, enhances the work-flow and reduces the costs of large and small scale expression profiling studies.

## Summary of the Invention

The present invention provides a method for real time analysis of cultured cells and their molecular content. More precisely, the present invention provides a method to monitor the cellular reaction of cells to certain stimuli in real time in order to figure out a reasonable time point to perform an analysis of the molecular content of said cells.

One aspect of the present invention concerns a method for the time resolved analysis of cells comprising

a) providing a cell type on a sensoric surface,

b) providing a compound,

c) monitoring a time dependent phenotypical signature of said cells in real time using said sensoric surface after treatment of said cells with said compound,

d) comparing in real time said time dependent phenotypical signature monitored in step c) with a predetermined phenotypical signature either obtained with the same or similar cell type or obtained with the same or similar component, said predetermined phenotypical signature comprises at least a first characteristic feature, and

e) analyzing at least a fraction of the molecular content of said cells on said sensoric surface upon occurrence of said characteristic feature in said time dependent phenotypical signature monitored in step c).

Any kind of cells may be used throughout the present invention provided that said cells are at least partially adherent to the sensoric surface and have the tendency to form a confluent cell monolayer. In order to enhance the adhesion of cells, the sensoric surface may be coated with certain materials, if this is necessary depending on the cells that should be analyzed with the method of the present invention.

Because the sensitivity of sensoric surfaces will decline with distance from the surface, the method of the present invention has only limited applicability for non-adherent cells. If non- or weakly adherent cells should be analyzed the sensoric surface has to be coated with materials enhancing the binding to the surface. These materials are known in the art and include positively charged substances like poly-L-lysine, collagens, gelatin, or fibronectin.

With respect to compounds all chemicals may be used that have an impact on the cells, whereas the impact must at least partially result in a change of cell morphology, cell adhesion, division rate and/or cell adhesion, because these kind of changes can be monitored by the sensoric surface in real time.

The phrase "time dependent phenotypical signature" is used throughout the present invention to emphasize that the sensoric surface is used to monitor the behavior of the cells in response to a treatment with a certain compound on a phenotypical level. But the person skilled in the art will appreciate that certain monitored phenotypical changes of the cells may have their basis on a genetic level.

Even though cells of a population may respond different to a certain compound, it is expected that their response is at least similar on a superior level, such as the compound will affect adhesion, impact cell division or cause cell death. Therefore, the time dependent phenotypical signature of cells is used to evaluate a suitable time point for further analysis of the cells by searching for similarities.

The suitable time point for further analysis of the cells is identified by monitoring the phenotypical signature in real time, looking for a characteristic feature. Consequently, it is necessary to know said characteristic features prior to the actual experiment. Said known characteristic features are part of the so called predetermined phenotypical signature of the present invention and the predetermined phenotypical signatures represent the control measurements of the present invention.

In order to have comparability between the actual experiment and the predetermined phenotypical signature it is advantageous that either the cell type or the compound is the same or at least similar.

The present invention is based on scanning the phenotypical signature of cells for characteristic features that provide an indication for respective changes e.g. on the genetic level of said cells. The person skilled in the art will of course recognize that not all phenotypical changes will have a genetic reason and that for certain situations there might be a certain time gap between the genetic change and the phenotypical change.

These fundamental principals need to be considered in order to profit from the analytic power of the present invention. Suppose a certain phenotypical change has its reason in a genetic change, but until said characteristic phenotypical change is detected, the genetic level may have changed in the meantime, too. Consequently, the genetic levels measured upon detection of a characteristic phenotypical signature may be different from the genetic level at the time point the phenotypical change was triggered.

Another aspect of the present invention is a kit for the time resolved gene expression analysis of cells according to the present invention comprising

a) a lysis buffer, which optionally comprises a chaotropic agent,

b) reagents to perform a gene expression analysis of said cells based on PCR, and

c) a database comprising a set of predetermined phenotypical signatures together with a corresponding time dependent predetermined gene expression profile.

Such a kit according to the present invention comprises all components that are necessary to perform a time resolved gene expression analysis of cells, namely the reagents for cell lysis as well as for gene expression analysis based on PCR amplification and a database comprising predetermined phenotypical signatures linked to the corresponding time dependent predetermined gene expression profiles.

With such a kit, the person skilled in the art having the necessary hardware equipment such as a cell analyzer (e.g. the xCELLigence® system of Roche Diagnostics, Cat.No. 05228972001), a sample preparation device (e.g. the MagNA pure® systems of Roche Diagnostics, e.g. Cat. No. 03731146001 or Cat. No. 05197686001) and a PCR device (e.g. the LightCycler® systems of Roche Diagnostics, e.g. Cat. No. 04484495001 or Cat. No. 05015278001) can perform the method for the time resolved analysis of cells according to the present invention.

Yet another aspect of the present invention is a system to perform the method according to the present invention comprising

a) a cell analyzer for monitoring a time dependent phenotypical signature of cells in real time,

b) a database comprising a set of predetermined phenotypical signatures together with a corresponding time dependent predetermined molecular profile, said predetermined phenotypical signatures each comprises at least a first characteristic feature, and

c) a computer program to compare said time dependent phenotypical signature monitored by the cell analyzer in real time with the predetermined phenotypical signature of said database.

A standard cell analyzer such as the xCELLigence® system of Roche Diagnostics GmbH can be transformed to a system according to the present invention by combining the cell analyzer with a database comprising a plurality of predetermined phenotypical signatures and a suitable computer program that performs the comparison of the actual experiment with the database signatures.

## Detailed Description of the Invention

One aspect of the present invention concerns a method for the time resolved analysis of cells comprising

a) providing a cell type on a sensoric surface,

b) providing a compound,

c) monitoring a time dependent phenotypical signature of said cells in real time using said sensoric surface after treatment of said cells with said compound,

d) comparing in real time said time dependent phenotypical signature monitored in step c) with a predetermined phenotypical signature either obtained with the same or similar cell type or obtained with the same or similar component, said predetermined phenotypical signature comprises at least a first characteristic feature, and

e) analyzing at least a fraction of the molecular content of said cells on said sensoric surface upon occurrence of said characteristic feature in said time dependent phenotypical signature monitored in step c).

A preferred method according to the present invention is a method, wherein upon occurrence of said characteristic feature in step e) at least a fraction of cells is removed from said sensoric surface in order to perform said analysis of the molecular content.

Depending on the analysis procedure that will be used after a characteristic feature occurred, it may be necessary to remove a fraction or the entire population of the cells from the sensoric surface. But on the other hand, there are other analysis procedures that may be performed directly on the sensoric surface. Such analysis procedures comprise e.g. optical or electrical techniques.

If cells are removed from the sensoric surface, this will have an effect on the signal the sensoric surface produces and consequently, the real time monitoring of the time dependent phenotypical signature of the cells can not be continued. Therefore, if the cell monitoring is performed in order to search for more than one event, each represented by its characteristic feature, the respective number of identical assays needs to be performed. In other words, one assay is provided for the continuous monitoring of the cells and one assay is provided for each of the expected events that need an additional analysis based on the extraction of cells.

Providing more than one assay is preferably done by arranging the plurality of assays in separate wells of multiwell plates, said multiwell plates may be used in 6-well, 24-well, 96-well, 384-well, or 1536-well format.

In case of an additional analysis that is performed directly on the sensoric surface, two different scenarios are possible. If the additional analysis is invasive, the situation is the same like in case of the removal of cell. On the other hand, if the additional analysis is non-invasive, it may be possible that the real time monitoring of the cells can be continued under certain circumstances. E.g. in order to obtain a monitoring without interruption, the additional analysis technique must be performed in addition to said monitoring.

Alternatively, a sensoric surface may be provided that has a region without sensoric activity and therefore, the removal of cells for the subsequent analysis may be performed with cells from this region of the sensoric surface without effecting the real time monitoring of cells on the sensoric part of the surface.

In a more preferred method according to the present invention, said removed fraction of cells is a single cell, a certain number of cells or the entire population of cells.

The number of cells that need to be removed from the sensoric surface for subsequent analysis depends on the subsequent analysis technique. E.g. the person skilled in the art knows that PCR is possible using nucleic acids from only a single cell. For instance, Bengtsson, M., et al., Genome Research 15 (2005) 1388-1392, have studied the expression of multiple genes in individual mouse pancreatic islet cells by reverse transcriptase quantitative real-time PCR (q-RT-PCR). This technique affords superior sensitivity, accuracy, and dynamic range compared with that of alternative methods for gene expression analysis. Schlieben, S., et al., Bio-Nobile Oy, Technical Note Molecular Biology TN41000-003 (2004) have previously described methods for the mRNA isolation from individual limited cell samples and single cells.

In another more preferred method according to the present invention, said removed fraction of cells is lysed prior to analyzing at least a fraction of the molecular content.

In most of the cases it will be necessary to perform an additional lysis step in order to analyze the molecular content of the extracted cells.

After the lysis, it may be necessary to separate the fraction of the molecular content to be analyzed from the remainder of the cell. For this separation step the person skilled in the art may apply different techniques including but not limited to filtration, centrifugation, phase separation, electrophoretic, absorption or chromatographic techniques.

Cells may also be disrupted by enzymatic or physical treatment, e.g. by sonification or other mechanical treatment to liberate molecular content to be analyzed from the remainder of the cell. Another method to disrupt cells is to add a hypoosmolaric solution that leads to swelling and final explosion of the cells.

Enzymatic or physical treatment e.g. by sonification or other mechanical treatment can also be applied to first remove the cells from the sensoric surface before the molecular content to be analyzed is liberated from the remainder of the cells. In certain cases it may even be possible to analyze intact cells e.g. when the molecular content to be analyzed is present at the cell surface.

In yet another more preferred method according to the present invention, said extraction of cell is performed after adding a lysis reagent to the sensoric surface.

In this embodiment said lysis reagent is used to liberate and dissolve the cells in order to analyze the fraction of the molecular content of said cells. This lysis reagent may be a chemical e.g. a detergent or an enzyme e.g. a Lipase that is able to disintigrate the cell membrane. The steps of adding the lysis reagent to the sensoric surface and to extract the cell lysis from the sensoric surface can be performed by manual or automated pipetting.

Preferred methods according to the present invention are those that can be performed in an automated fashion based on simple automated pipetting steps. In more detail, a pipetting robot will automatically add a lysis reagent to the sensoric surface upon occurrence of said characteristic feature in said time dependent phenotypical signature and afterwards a pipetting robot will automatically extract the lysed cells from said sensoric surface.

Another preferred method according to the present invention comprises after step

e) a further step

f) comparing said fraction of the molecular content determined in step e) with a predetermined fraction of the same or similar molecular content obtained with either the same or similar cell type or the same or similar compound at the characteristic feature of the corresponding predetermined phenotypical signature.

In this preferred embodiment of the present invention the determined molecular content is compared with predetermined molecular content, said predetermined molecular content is linked to the characteristic feature of the predetermined phenotypical signature. If a predetermined phenotypical signature has more than one characteristic feature, a predetermined molecular content can be linked to each of said characteristic features.

Based on the fundamental principals outlined before, namely that there might be a certain time gap between e.g. the genetic change and the phenotypical change, at least two different cases need to be considered, if a molecular content is obtained upon occurrence of a characteristic feature.

If the molecular content obtained upon occurrence of a characteristic feature is used for the characterization of the cells and/or the compound, it is preferred to perform the experiments such that also for the time prior to occurrence of the characteristic feature molecular content is obtained. This can be done e.g. by performing a certain amount of assays in parallel, wherein each assay is started at a different time. If a characteristic feature occurs in the assay started first, the molecular content is not only obtained for this assay, but also for the other assays that started later in time. With this experimental design it is possible to obtain the molecular course prior to the occurrence of certain phenotypical signature, wherein a certain time resolution can be provided by the number of assays and time interval between said assays.

Alternatively, it is possible to link the characteristic feature of the predetermined phenotypical signature with the corresponding predetermined molecular content obtained at the characteristic time point, as well as with additional predetermined molecular contents corresponding to other time points prior to the occurrence of the signature. In this embodiment, the occurrence of a phenotypical signature and the accordance of the obtained molecular content provide also the information about the molecular course history based on the predetermined molecular contents.

In cases where the molecular content does not change between the initial trigger of the phenotypical signature and the detection of said phenotypical signature, the above mentioned experimental setups are not necessary and the molecular content obtained upon occurrence of the signature can directly be used for the intended characterization.

In another preferred method according to the present invention, said cells are cultured on said sensoric surface in step a).

In general, there a two different alternatives to provide cells on the sensoric surface, namely to place cultured cell on said sensoric surface or to seed only a small number of cells to produce a cell culture on said sensoric surface.

If only one or a small number of cells are added to said sensoric surface, it is possible to monitor the growth phase of the cells. On the other hand, placing cultured cells on the sensoric surface offers the advantage that the experiment can be started earlier.

In yet another preferred method according to the present invention, said time dependent phenotypical signature of said cells is monitored in real time for a certain time prior to the treatment of said cells with a compound.

Monitoring the time dependent phenotypical signature in real time prior to the treatment with the compound offers the opportunity to verify the initial status of the cells and to obtain a reference value to monitor relative changes of the phenotypical signature.

Moreover, monitoring the time dependent phenotypical signature in real time prior to the treatment with the compound offers the additional opportunity to verify a suitable time point for said treatment. Consequently, in this embodiment of the invention the monitored time dependent phenotypical signature of said cells is compared in real time with predetermined phenotypical signatures comprising at least a first characteristic feature indicating the time point for the treatment of said cells with certain compound.

For example, by monitoring the time dependent phenotypical signature in real time prior to the treatment with the compound, it is possible to determine a suitable time point for a treatment of the cells with a compound, if e.g. it is explicitly required to apply said compound in the growth phase or in the plateau phase of cells.

With respect to the sensoric surface several different combinations of surface and analytical technique are applicable within the scope of the present invention. In general, any surface sensitive technique providing the opportunity to detect changes in the cellular layer covering the surface may be used. Such surface sensitive techniques are e.g. surface plasmon resonance (SPR) using gold substrates, evanescent field techniques using optical transparent substrates or electric techniques such as voltametry or impedance measurements.

For SPR or evanescent field techniques homogeneous surfaces can be used as sensoric surface. For SPR e.g. a glass slide coated with a homogeneous gold layer on one side may be used.

In still another preferred method according to the present invention, said time dependent phenotypical signature is measured using an electric technique.

Even though, the person skilled in the art will recognize that an electric set-up is possible with only a homogeneous sensoric surface as the first electrode and another external electrode as reference electrode, it is of advantage to provide structured sensoric surfaces.

Therefore, yet another preferred method according to the present invention is a method, wherein said sensoric surface is a surface comprising an electrode.

In a more preferred method according to the present invention, said sensoric surface is a surface comprising an array of electrodes, preferably said sensoric surface is a surface comprising an array of interdigitated gold electrodes.

Interdigitated electrodes may provide a large sensoric area on a given surface, whereas such an interdigitated electrode structure consists of two electrodes, each electrode has a connection pad with a certain number of elongated structures and said elongated structures interleave each other to form the interdigitated structure. Different geometries of interdigitated electrodes are possible, e.g. a comb-like geometry, whereas each elongated structure is simply rectangular or comprises additional features along the elongated structure such as circles, bars or diamonds (see figure 15 of WO 2004/010102). Alternatively, the elongated structure can be provided in a wave-like structure (see figure 11 or 13 of WO 2007/085353). Moreover, a concentric electrode structure is possible, too (see figure 15F of WO 2004/010102).

With respect to the electrode material gold is a preferred material, because it is inert, non-toxic for cells and allows adherence as well as growth of cells.

In another more preferred method according to the present invention, said electric technique is impedance measurement.

The use of impedance measurements for cellular analysis is well known to the person skilled in the art and therefore, the general principals are not explained here, but it is referred to state of the art documents such as US 7,192,752.

Briefly, due to the presence of cells on the sensoric surface the electrical properties of the interface between the electrode surface and the buffer solution changes, whereas said changes can be detected by impedance measurement. At the electrode/electrolyte interface there are mainly two different surface phenomena that are affected by the presence of cells, namely the charge transfer across the interface as well as its dielectric behavior and both phenomena occur at different frequencies of the applied ac voltage. Consequently, said phenomena can be separated in the frequency space of the applied ac voltage.

The two surface phenomena introduced above will change the measured impedance between two extreme values, namely the value of a bare electrode surface on the one side and an electrode surface completely covered with cells on the other.

Therefore, a preferred method according to the present invention is a method, wherein said time dependent phenotypical signature is a measure for the cell coverage of said electrodes.

Said cell coverage of the sensoric surface can be altered by a plurality of effects, e.g. a change in cell number (increasing by cell division or decrease by cell death), a change in cell size (due to uptake or release of electrolyte), a change in cell morphology (switch from a platelet to a round configuration) and/or a change of cell adhesion to the sensoric surface.

All the above mentioned effects that can be monitored by impedance measurement and are summarized as the phenotypical signature of a certain cell type in response to a certain stimulus.

Such phenotypical signatures are characteristic for a respective cell/stimulus pair and with each experiment a phenotypical signature is obtained that can be stored to be used for the successive experiments as a predetermined phenotypical signature.

In another preferred method according to the present invention, said predetermined phenotypical signature is obtained from a data base.

Alternatively, it is also possible to perform the method according to the present invention without a database comprising predetermined phenotypical signatures, namely to perform a reference experiment prior or parallel to the actual experiment using e.g. either the cells or the compound of said actual experiment.

In yet another preferred method according to the present invention, said predetermined phenotypical signature is obtained by performing the following steps

i) providing a cell type on a sensoric surface,

ii) providing a compound,

iii) monitoring a time dependent phenotypical signature of said cells in real time using said sensoric surface after treatment of said cells with said compound.

In the actual experiment, the monitored phenotypical signature is compared either to a certain predetermined phenotypical signature or to a certain number of predetermined phenotypical signatures in real time to observe the occurrence of certain characteristic features. Said characteristic features are an indication of the cellular background effects that provide the observed phenotypical response and trigger the subsequent analysis of the molecular content of the cells.

Throughout the present invention, a plurality of characteristic features is suitable as a trigger for the subsequent analysis of the molecular content of the cells, whereas the necessary correlation between the predetermined and the monitored phenotypical signature to identify a match may be defined by the user of the method.

In a preferred method according to the present invention, said characteristic feature of said predetermined phenotypical signature is a discontinuous change of said time dependent course.

In a more preferred method according to the present invention, said discontinuous change is a change of the absolute value of said time dependent course.

In another more preferred method according to the present invention, said discontinuous change is a change of the slope of said time dependent course.

In order to identify said discontinuous change of said time dependent course, it is possible to obtain the first or higher order derivative of said time dependent course. A person skilled in the art will recognize that this procedure may simplify the identification of discontinuous changes.

In another preferred method according to the present invention, said characteristic feature of said predetermined phenotypical signature is reaching a threshold value of said time dependent course.

Such a threshold value may be defined e.g. as the doubling or the bisection of an initial reference value.

In yet another preferred method according to the present invention, said characteristic feature of said predetermined phenotypical signature is a plateau phase of said time dependent course.

Such a plateau phase of said time dependent course may be defined e.g. by a certain time interval without changes of the time dependent course. The plateau criterion is preferably defined via a certain percentage that the time dependent course is allowed to change during the respective time interval.

In still another preferred method according to the present invention, said characteristic feature of said predetermined phenotypical signature is an increase after a plateau phase of said time dependent course.

In another preferred method according to the present invention, said characteristic feature of said predetermined phenotypical signature is a decrease after a plateau phase of said time dependent course.

These two characteristic features have two requirements that need to be fulfilled in order to trigger the respective subsequent molecular analysis. First, the time dependent course must be constant (within defined boarders) for a certain amount of time and afterwards, an increase/decrease of the time dependent course must occur, whereas said increase/decrease is preferably defined as a percental increase/decrease.

Throughout the present invention different kinds of analysis techniques are possible to determine at least a fraction of the molecular content of said cells on said sensoric surface upon occurrence of said characteristic feature in said time dependent phenotypical signature. In general, there are two different basic situations for such an analysis, namely an analysis on said sensoric surface or an external analysis after removal of cells from said sensoric surface.

In a preferred method according to the present invention, said analysis in step e) is a gene expression analysis.

For such a gene expression analysis it is necessary to perform a lysis of the cells prior to the analysis.

In case of adherent cells, the lysis protocols as used in the art require an additional trypsination step, which means that in order to detach the adherent cells from the solid support the cell culture is incubated with an appropriate buffer solution containing Trypsin-EDTA which is commercially available (e. g. Invitrogen Cat. No: 25200 056, Genaxxon Cat. No: 4260.0500).

The biological sample preferably consists of adherent eukaryotic cells, i.e. the cells are cultivated and grow by being attached to a solid support that is part of a cultivation vessel. For the inventive method according to the present invention, any type of cultivation vessel can be used provided that said cultivation vessel can be equipped with a sensoric surface. Examples, which however, are not limiting, the scope of the present invention are Petri dishes or cultivation bottles having an inner surface that is suited to be a solid support for the sensoric surface and for the growth of cells. Other examples for cultivation vessels are microtiter plates in the 6-well, 24-well, 96-well, 384-well, or 1536-well format as they are commonly used in the art.

In case the method according to present invention is performed in such microtiter plates, it is possible to cultivate, lyse and reverse transcribe multiple samples in parallel. More precisely, cell cultivation, cell lysis, dilution, any addition of additives and the reverse transcriptase reaction are carried out in the same reaction vessel. Therefore, this embodiment of the method according to the present invention is particularly useful for high throughput analyses of multiple samples of adherent cells within an automated process. If the reaction vessels are arranged together in the form of a 24, 96, 384 or 1536 well microtiter plate according to standards that are established in the art, the lysis reagent, the various additives and the reagents necessary for performing a Reverse Transcriptase reaction can be added to the samples by liquid handling robotic instruments. Note that this embodiment of the method according to the present invention does not require detachment of the cells from the solid support by trypsin, because the cells are directly lysed in situ. More details about this strategy can be found in patent application EP 08/013816.7 filed August 1, 2008.

After nucleic acids are extracted from the cells, there are mainly two different analysis techniques available to perform the gene expression analysis.

In a preferred method according to the present invention, said gene expression analysis is based on PCR.

The principals of PCR reaction are familiar to the person skilled in the art, namely that a polymerase and a specific pair of amplification primers, which is designed to allow for the detection of a specific nucleic acid species, are necessary.

More preferably, said gene expression analysis is based on real time PCR. Such a monitoring in real time is **characterized in that** the progress of said PCR reaction is monitored in real time. Different detection formats are known in the art. The below mentioned detection formats have been proven to be useful for PCR and thus provide an easy and straight forward possibility for gene expression analysis:

a) TaqMan Hydrolysis probe format:

A single-stranded Hybridization Probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured. TaqMan probe assays are disclosed in detail in US 5,210,015, US 5,538,848, and US 5,487,972. TaqMan hybridization probes and reagent mixtures are disclosed in US 5,804,375.

b) Molecular Beacons:

These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

c) FRET hybridization probes:

The FRET Hybridization Probe test format is especially useful for all kinds of homogenous hybridization assays (Matthews, J.A., and Kricka, L.J., Analytical Biochemistry 169 (1988) 1-25). It is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. Alternatively to monitoring the increase in fluorescence of the FRET acceptor component, it is also possible to monitor fluorescence decrease of the FRET donor component as a quantitative measurement of hybridization event.

In particular, the FRET Hybridization Probe format may be used in real time PCR, in order to detect the amplified target DNA. Among all detection formats known in the art of real time PCR, the FRET-Hybridization Probe format has been proven to be highly sensitive, exact and reliable (WO 97/46707; WO 97/46712; WO 97/46714). As an alternative to the usage of two FRET hybridization probes, it is also possible to use a fluorescent-labeled primer and only one labeled oligonucleotide probe (Bernard, P.S., et al., Analytical Biochemistry 255 (1998) 101-107. In this regard, it may be chosen arbitrarily, whether the primer is labeled with the FRET donor or the FRET acceptor compound.

d) SybrGreen format

It is also within the scope of the invention, if real time PCR is performed in the presence of an additive according to the invention in case the amplification product is detected using a double stranded nucleic acid binding moiety. For example, the respective amplification product can also be detected according to the invention by a fluorescent DNA binding dye which emits a corresponding fluorescence signal upon interaction with the double-stranded nucleic acid after excitation with light of a suitable wavelength. The dyes SybrGreenI and SybrGold (Molecular Probes) have proven to be particularly suitable for this application. Intercalating dyes can alternatively be used. However, for this format, in order to discriminate the different amplification products, it is necessary to perform a respective melting curve analysis (US 6,174,670).

In another preferred method according to the present invention, said gene expression analysis is based on the read-out of DNA hybridization arrays.

A hybridization array comprises a surface with a certain number of different sites, to each of said sites a plurality of oligonucleotides having a certain sequence are coupled. Said coupled oligonucleotides are suitable to hybridize to complimentary nucleotides of a liquid sample, if the hybridization array is in contact with said liquid sample under hybridization conditions.

The read out of the hybridization array in terms of hybridization sites can be performed e.g. by detection of a label that is attached to the nucleic acids of the sample. Consequently, the fluorescence signal of a certain array site indicates that the complementary nucleotide is present in the liquid sample.

In yet another preferred method according to the present invention, said analysis in step e) is a protein analysis, preferably a protein expression analysis or a protein modification analysis.

In a more preferred method according to the present invention, said protein expression analysis is based on Western blotting or large scale proteomics analysis.

A suitable analytical technique for the large scale proteomics embodiment of the present invention is e.g. mass spectrometry.

In another more preferred method according to the present invention, said protein modification analysis is based on a phosphorylation analysis.

The above mentioned protein analysis is based on either, uptake of radioactively labeled molecules into living cells, e.g. phosphorus-32, and quantification of their incorporation into protein(s) of interest by special imaging techniques, such as a phosphor imager, by Western Blotting applying modification-specific antibodies (e.g. phosphorylation-specific antibodies) or on particular mass spectrometry techniques that are able to quantify the extent of modification as well as to identify the specific site of modification within a protein.

Another aspect of the present invention is a kit for the time resolved gene expression analysis of cells according to the present invention comprising

a) a lysis buffer, which optionally comprises a chaotropic agent,

b) reagents to perform a gene expression analysis of said cells based on PCR, and

c) a database comprising a set of predetermined phenotypical signatures together with a corresponding time dependent predetermined gene expression profile.

A preferred kit according to the present invention is a kit, wherein said reagents to perform a gene expression analysis comprise reagents for the separation of nucleic acids from the cell debris.

Another preferred kit according to the present invention is a kit, wherein said reagents to perform a gene expression analysis comprise a set of primers and probes for the PCR based analysis of the expression of a certain set of genes.

The reagents necessary to perform a sample preparation and a PCR based analysis are known to the person skilled in the art and are commercially available, e.g. from Roche Diagnostics GmbH. Therefore, no details are provided here, but it is referred to the appropriate literature.

Yet another preferred kit according to the present invention is a kit, wherein said database is a database on a portable data storage medium.

The predetermined phenotypical signatures together with a corresponding time dependent predetermined gene expression profile are provided as part of the kit according to the present invention, said signatures and profiles are structured in database. The database can be provided on several kinds of storage media, e.g. CDs, memory sticks or hard discs.

Still another preferred kit according to the present invention is a kit, wherein said database is a database on a server and the kit comprises a link to said server.

In this alternative of the kit according to the present invention, the database as such is not provided as part of the kit, but only information about where and how the database can be accessed. The access to the database on a server can be realized in at least two different ways, namely the link is provided to download the entire database to the computer of the kit user via the internet or the link is provided to perform the comparison of the monitored time dependent phenotypical signature with the predetermined phenotypical signature within the database on the server computer. Within a second alternative, the database information is not transferred to the kit user, but the monitored signals are transferred to the server via the internet and the results of the comparison are subsequently transferred back to the kit user.
Yet another aspect of the present invention is a system to perform the method according to the present invention comprising

a) a cell analyzer for monitoring a time dependent phenotypical signature of cells in real time,

b) a database comprising a set of predetermined phenotypical signatures together with a corresponding time dependent predetermined molecular profile, said predetermined phenotypical signatures each comprises at least a first characteristic feature, and

c) a computer program to compare said time dependent phenotypical signature monitored by the cell analyzer in real time with the predetermined phenotypical signature of said database.

As mentioned before, a suitable cell analyzer is the xCELLigence® system of Roche Diagnostics GmbH. Because in most of the cell analysis applications it is necessary to have reference assays as well as additional assays to monitor the time dependent phenotypical signature for more than one characteristic feature, it is preferred to provide a cell analyzer that is suitable to perform a plurality of assays in parallel.
Such a parallelization is preferably realized based on multiwell plates. The xCELLigence® system of Roche Diagnostics GmbH is manufactured to work with 96 well plates enabling the user to perform 96 assays in parallel or even of 6 separate 96 well plates in parallel.
A preferred system according to the present invention further comprises an extraction device, said extraction device is arranged such that a fraction of said cells monitored in real time is extracted upon the corresponding indication from said computer program.
As mentioned before, for certain analysis techniques it may be necessary to remove at least part of the cells from the assay.
The person skilled in the art will know about options to isolate small numbers of purified cells from complex cellular samples such as micromanipulation, fluorescence-activated cell sorting or laser microdissection and said techniques are e.g. described in the following review articles: Burgemeister, R., "New aspects of laser microdissection in research and routine", J Histochem Cytochem. 53(3) (2005) 409-12 and Baech, J., and Johnsen, HE, "Technical aspects and clinical impact of hematopoietic progenitor subset quantification", Stem Cells 18 (2000) 76-86.
Alternatively, it is possible to add lysis reagent directly to the sensoric surface using pipetting robots and extracting the lysed cells afterwards using also a pipetting robot. Consequently, this approach does not use a fraction of the cells on the sensoric surface, but all cells are used for the subsequent analysis.
Another preferred system according to the present invention further comprises a separation device, said separation device is arranged such that the molecular content from said cells monitored in real time is separated from the cell debris upon the corresponding indication from said computer program.
As mentioned before, suitable separation device are commercially available. For the isolation of nucleic acids from cell samples e.g. the MagNA Pure Compact system (Cat. Nr. 03731146001) or the MagNA Pure LC 2.0 system (Cat. Nr. 05197686001) of Roche Diagnostics GmbH can be used.
Yet another preferred system according to the present invention further comprises an analysis device, said analysis device is arranged such that a molecular profile of said cells monitored in real time is measured upon the corresponding indication from said computer program.
In a more preferred system according to the present invention, said analysis device is a PCR device, preferably a real-time PCR device.
As mentioned before, suitable analysis devices for the nucleic acid content of samples are commercially available such as the LightCycler® 1.5 (Prod. Nr. 04484495001), the LightCycler® 2.0 (Prod. Nr. 03531414001) or the LightCycler® 480 (Prod. Nr. 05015278001 for the 96-well version and Prod. Nr. 05015243001 for the 384-well version).
The following examples, sequence listing and figures are provided to aid the understanding of the present invention, the true scope of which is set forth in the appended claims. It is understood that modifications can be made in the procedures set forth without departing from the spirit of the invention.

## Description of the Figures

**Figure 1** Plot of Normalized Cell Index (CI) values for the entire course of the RTCA Paclitaxel experiment with MCF7 cells together with a reference curve (solid line).

**Figure 2** Column diagram demonstrating Paclitaxel-induced gene expression regulation in MCF7 cells at different time points.

**Figure 3** Cell growth curves of HT29 cells, whereas the Cell Index value was normalized at the time point of paclitaxel addition. A) Cell growth profile shows the initial cell attachment and logarithmic growth phase. The time point of treatment is indicated by the black solid line (paclitaxel lower curve, DMSO middle curve or medium only upper curve). B) The time points of paclitaxel addition (black solid line) and RNA Isolation (triangles) are indicated. The Cell Index for the wells with paclitaxel treated (lower curve) cells is almost zero 24 hours after treatment.

**Figure 4** Cell Index recorded during the first four hours after paclitaxel treatment compared with the WST-1 data obtained after one, two and four hours indicating the necessity of RNA analysis at an early time point. Treatment with paclitaxel was set to the time point zero.

**Figure 5** Ratio of gene expression of paclitaxel-treated sample to control (DMSO) using the RealTime Ready Human Apoptosis Panel 96 calculated and plotted for time points 1 hour (A), 2 hours (B), 4 hours (C) and 24 hours (D) after paclitaxel treatment.

**Figure 6** Selection of genes which expression levels have been significantly altered (>4 times)

**Figure 7** Ratio of gene expression of paclitaxel-treated sample to control (DMSO) using the RealTime Ready Human Cell Cycle Panel 96 calculated and plotted for the time points 1 hour (A), 2 hours (B), 4 hours (C) and 24 hours (D) after paclitaxel treatment.

## Example 1

Paclitaxel is a compound with anti-neoplastic activity, originally extracted from the Pacific yew tree Taxus brevifolia. It belongs to the group of tubulin-binding agents, which can be distinguished into microtubule-destabilizing agents, like vinca alkaloids, colchicine, podophyllotoxin and nocodazole, as well as microtubule-stabilizing agents, including taxanes, epothilones, discodermolide and eleutherobin. Taxanes bind to a special side on β-tubulin that is accessible for the drug only in assembled tubulin polymers. In this way it prevents the disassembly of tubulin filaments and the generation of unusually stable and functionally disrupted microtubules. But microtubule dynamics are an essential prerequisite for the disassembly of the interphase microtubule network and the subsequent build-up of the mitotic spindle. The lack of a functional mitotic spindle activates the mitotic spindle checkpoint, which consequently arrests cells in the metaphase of mitosis and thus corroborates cell division (McGrogan, BT, et al., Biochimica et Biophysica Acta 1785 (2008) 96-132; Jordan, M., A, and Wilson, L., Curr Opin Cell Biol 10 (1998) 123-130; Dumontet, C., and Sikic, B., I., J. Clin. Oncol. 17(3)(1999) 1061-1070). Nevertheless, anti-mitotic compounds, like Taxol, are proposed to interfere with mitosis, but also affect microtubules in interphase cells, e.g. altering neurite morphogenesis as well as adhesion and locomotion properties of cells.

It has been described that at moderate Paclitaxel concentrations the mechanism of drug action in inhibiting cell proliferation and killing tumor cells is mainly due to stabilizing spindle dynamics rather than excessive polymerization of tubulin (Jordan, M., A, and Wilson, L., Curr. Opin. Cell. Biol. 10 (1998) 123-130; Dumontet, C., and Sikic, B., I, J. Clin. Oncol. 17(3)(1999) 1061-1070). Paclitaxel was known to be toxic for hundreds of years, its benefit, however, was only discovered in 1964. From then on it was used as a drug in chemotherapy and was first clinically applied in 1993. Nowadays, Paclitaxel is produced chemically and has become a standard in oncologic therapy of advanced ovarian carcinoma and metastatic breast cancer. Incorporation occurs via intravenous infusion. The uptake is followed by non-linear pharmacokinetics - the drug gets metabolized in the liver and excreted predominantly by the bile. Because of its lipophilic character, Paclitaxel is easily absorbed into cells. The absorption and the mitotic block are not restricted to tumor cells, but affect also the cell cycle of frequently dividing healthy cells. Due to its various side effects, including alopecia, myelosuppression, gastrointestinal symptoms and febrile neutropenia, new forms of Paclitaxel, e.g. connected to Albumin, have been developed to avoid these sorts of hypersensitivity. Treatment occurs in cycles interrupted by application-free periods (McGrogan, B., T, et al., Biochimica et Biophysica Acta 1785 (2008) 96-132; Dumontet, C., and Sikic, B., I, J. Clin. Oncol. 17(3)(1999) 1061-1070).

The mitotic arrest persists for varying lengths of time, depending on cell type and drug dose. In addition, the concomitant cellular effects in response to treatment with an anti-mitotic agent may vary. On one hand, cells can undergo sustained or chronic mitotic arrest until the drug is cleared by diffusion and/or removal from cells through active pump-out via so-called multi-drug resistance transporters. This enables cells to survive and continue dividing as diploid cells. On the other hand, cells can die via apoptosis directly during the time of the mitotic arrest. Most cells override the mitotic spindle checkpoint signaling, pass through mitosis and divide with unequal segregation of sister chromatids - generating cells with different content of genomic DNA. These cells often become apoptotic and die because of aneuploidy during the following round of the cell cycle. In addition, adaptation and so-called "mitotic slippage" can occur when cells exit mitosis without engaging in metaphase and without cytokinesis, producing tetraploid, multi-nucleated cells. Such cells can survive, enter G1-phase of the next cell cycle and continue dividing as tetraploid cells, but die of apoptosis during later cell division cycles. Eventually, these cells immediately exit G1-phase and become senescent and/or apoptotic (McGrogan, B., T, et al., Biochimica et Biophysica Acta 1785 (2008) 96-132; Jordan, M.,A., and Wilson, L., Curr Opin Cell Biol 10 (1998) 123-130; Dumontet, C., and Sikic, B., I., J. Clin. Oncol. 17(3)(1999) 1061-1070).

The biochemical events leading to drug resistance or apoptosis upon Paclitaxel treatment are complex, little understood and may be concentration-dependent as well as cell type-specific. However, it is clear that apart from the direct effect on microtubules and ultimate changes in cell morphology and adhesion, the drug may induce profound gene expression changes during the time of drug exposure, leading to alterations in expression levels of proteins involved in apoptosis, mitotic slippage as well as drug resistance (Dumontet, C., and Sikic, B., I., J. Clin. Oncol. 17(3)(1999) 1061-1070).

In this example, we have investigated gene expression profiles of human MCF-7 breast cancer cells (human Caucasian adenocarcinoma breast cancer cell line) obtained from ATCC (passage number: 15, cell number: 5000 cells per well), maintained in MEM (32360, Gibco) + 10% FCS (30-3702, PAN) + Na-Pyruvat (P04-43100, PAN) + Nonessential amino acids (P08-32100, PAN) at time points that are indicated by changes in cell characteristics as visualized by changes of impedance (cell indices) measurements upon drug addition. Accordingly, gene expression profiles were determined at 6 h, 24 h, 72 h and 147 h upon Paclitaxel administration. Hereby, we focused on a predefined set of genes the expression of which had been found to alter greatly in response to administration of Paclitaxel into mice bearing ovarian carcinoma xenografts and that had been obtained by cDNA microarray analysis (Bani, MR, et al., Molecular Cancer Therapeutics 3(2) (2004) 111-121). It includes genes involved in various biological functions such as cell cycle regulation and cell proliferation, apoptosis, signal transduction and transcriptional regulation, fatty acid and sterol metabolism and IFN-mediated signaling (Bani, M., R., et al., Molecular Cancer Therapeutics 3(2) (2004) 111-121). With respect to the two time points (6 h and 24 h upon drug administration) microarray studies had been performed by other groups and we were able to reproduce 70% of their results (Bani, M., R., et al., Molecular Cancer Therapeutics 3(2) (2004) 111-121). In addition, we determined mRNA levels at two additional time points (72 h and 147 h upon drug administration) during prolonged drug exposure of cells. Apart from the last time point (ca. 170 h) all time points correlate with a change in cell behavior and may be at least partially induced through expression changes of some of the investigated genes.

We have performed gene expression profiling experiments of MCF-7 cells treated with Paclitaxel for a period of approximately 150 h. Within this example the gene expression of 20 different genes (gene accession numbers in brackets) were monitored:

| | |
|---|---|
| HDAC3 (ENST00000305264.1) | GNAI1 (ENST00000078429.3) |
| ISG15 (ENST00000379389.2) | IFITM1 (ENST00000399815.1) |
| BNIP3 (ENST00000368636.1) | SLUG (ENST00000020945.1) |
| FOS (ENST00000303562.2) | ARF1 (ENST00000327482.2) |
| CDKN1A (ENST00000244741.2) | PIG8 (ENST00000278903.4) |
| CDC2 (ENST00000395284.1) | PLAB (ENST00000252809) |
| TOP2A (ENST00000269577.4) | MADH2/SMAD2 (ENST00000356825.3) |
| ATF2 (ENST00000392544.1) | SPRY4 (ENST00000344120.2) |
| LIPA (ENST00000336233.4) | IDI1 (ENST00000381344.2) |
| FDPS (ENST00000368356.1) | IGFBP5 (ENST00000233813.2) |

As reference genes the following housekeeping genes were used:

β-Actin (NM_001101.2)

β-Globin (ENST00000335295)
GAPDH (ENST00000229239)

We intended to determine and reproduce gene expression changes with time upon drug treatment, the majority of which had been observed and described before by others in an independent in vivo-study based on DNA microarray technique using ovarian carcinoma xenografts (Bani, M., R., et al., Molecular Cancer Therapeutics 3(2) (2004) 111-121; Boschke, C., B., et al., Uni Tübingen (2008)). The time points for our pharmacokinetic screens have been chosen depending on cellular changes in response to Paclitaxel-treatment which were monitored by impedance-based real time cell analysis using the xCELLigence® system. Independent from the quality and extent of these cellular changes we harvested non-treated controls and drug-treated cells at their appropriate time points (6, 24, 72 and 147 h), isolated the mRNA by means of the MagnaPure System, reverse transcribed the total mRNA into cDNA using a common Thermocycler, pooled and diluted the cDNA samples and amplified the predefined set of specific genes as well as house keeping genes in triplicates by q-RT-PCR making use of the LightCycler® 480 System. Necessary primer pairs were synthesized and tested for functionality in-house in combination with a bioinformatically determined UPL probe (data not shown). The LightCycler® software 1.5 allowed the relative quantification of the selected mRNA abundance under Paclitaxel-treated conditions with respect to the corresponding non-treated situation (reference). Results are corrected by the values determined for internal standard genes (stably expressed house keeping genes), like β-Actin, β -Globin and GAPDH.

**Procedure: Seeding, Growth, Treatment, Follow-up, Harvest and Lysis**

- Day 1: Time point 0 h: we added 100 μl medium to each well of the 96 well-E-Plate (E-Plate number: S/N: C10090 NT L/N: 080305, Roche) and performed the background measurement in the SP station (Single Plate xCELLigence® Instrument W380, serial number: 28-1-0712-1005-7; Software: SP1.0.0.0807, Roche), then we added 100 μl of the MCF-7 cell suspension (concentration: 50000 cells/ml = 5000 cells per well)
- we let cells settle and attach for 30 min at room temperature
- Day 1: Time point 0.7 h: E-Plate was put into SP station, impedance measurement started (every 15 min)
- Day 2: Time point 23 h: we paused the measurement and started the Paclitaxel treatment (Control: 0.1 % DMSO final concentration, compound treatment: 12.5 nM Paclitaxel in DMSO final concentration ; Paclitaxel obtained from Sigma-Aldrich, stock solution 50 μM in DMSO)
- Day 2: Time point 23.25 h: we restarted the measurement
- Day 2, Day 3, Day 5, Day 8 or 6, 24, 72 and 147 h upon drug treatment: Time point 29.5, 47.5, 95.5, 170.5 h: we harvested the complete population of control- and compound-treated cells, pelleted and lysed them in Magna Pure Ready-to-use lysis buffer (Roche)
- Lysates were stored at -80°C

**Procedure: RNA isolation**

- cell lysates (300 μl) of up to $1x10^6$ cells were thawed on ice
- mRNA isolation was carried out automatically by the Magna Pure Instrument (Roche)
- all buffers and reagents (capture buffer, wash buffer II, DNAse solution, wash buffer I, Streptavidin Magnetic Particles, elution buffer) were used and prepared according the MagNA Pure LC mRNA Isolation kit I (03004015001, Roche)
- and had to be warmed to room temperature
- volumes of buffers and reagents were calculated by the appropriate Instrument Software "mRNA I Cells"

- reagents and buffers were pipetted into Nuclease-free disposables (Eppendorf) outside the instrument and under a flow cabinet
- isolated mRNA (in 25 μl elution buffer) were constantly kept at 4°C and immediately reverse transcribed into cDNA

**Procedure: RT-PCR**

- mRNA was transcribed into cDNA
- Mastermixes were prepared according manufacturer instructions (final concentrations: 1x reaction buffer, 1mM dNTP-Mix (11814362001, Roche), 0.08 U Random primer (p(dN)6, 11034731001, Roche), 20 U RNAse inhibitor (03335492001, Roche), 10 U Transcriptor RT (03531287001, Roche), filled up with PCR-graded water, Roche)
- 15 μl Mastermix and 5 μl isolated mRNA were combined in PCR reaction tubes and put into the Thermocycler (PCR instrument, Thermocycler T3, serial number 35-51-02TC-04, 3003377 (Biometra))
- Program: Step 1: 10 min 25°C, Step2: 30 min 55°C, Step3: 5 min 85°C, Cooling: 4°C
- Samples of the same content were pooled and stored at -20°C

**Procedure: q-RT-PCR**

- cDNA samples thawed on ice
- cDNAs were diluted 1:5
- total PCR reaction volume: 20 μl including Primer-Probe Mix (final concentrations:

    0,5 uM forward and reverse primer:

    For genes:

        HDAC3 (SEQ ID NO:1, SEQ ID NO:2), GNAI1 (SEQ ID NO:3, SEQ ID NO:4), ISG15 (SEQ ID NO:5, SEQ ID NO:6), IFITMI(SEQ ID NO:7, SEQ ID NO:8), BNIP3(SEQ ID NO:9, SEQ ID NO:10), SLUG(SEQ ID NO: 11, SEQ ID NO:12), FOS(SEQ ID NO:13, SEQ ID NO:14), ARF-1 (SEQ ID NO:15, SEQ ID NO:16), CDKN1A (SEQ ID NO:17, SEQ ID NO:18), PIG8(SEQ ID NO:19, SEQ ID NO:20), CDC2(SEQ ID NO:21, SEQ ID NO:22), PLAB(SEQ ID NO:23, SEQ ID NO:24), TOP2A(SEQ ID NO:25, SEQ ID NO:26), MADH2/SMAD2 (SEQ ID NO:27, SEQ ID NO:28), ATF2 (SEQ ID NO:29, SEQ ID NO:30), SPRY4(SEQ ID NO:31, SEQ ID NO:32), LIPA(SEQ ID NO:33, SEQ ID NO:34), IDI1(SEQ ID NO:35, SEQ ID NO:36), FDPS(SEQ ID NO: 37, SEQ ID NO:38), IGFBP5(SEQ ID NO:39, SEQ ID NO:40)

    For housekeeping genes:

        β-Actin(SEQ ID NO:41, SEQ ID NO:42), β-Globin(SEQ ID NO:43, SEQ ID NO:44), GAPDH(SEQ ID NO: 45, SEQ ID NO:46)

    0.2 uM Universal probe library probes (Roche, genes with UPL probe numbers in brackets):

        For genes: HDAC3 (26), GNAI1 (53), ISG15 (76), IFITM1 (45), BNIP3 (84), SLUG (7), FOS (67), ARF1 (45), CDKN1A (82), PIG8 (6), CDC2 (79), PLAB (28), TOP2A (75), MADH2/SMAD2 (80), ATF2 (85), SPRY4 (17), LIPA (36), IDI1 (65), FDPS (15), IGFBP5 (77)

    For housekeeping genes: β-Actin (11),β-Globin (83), GAPDH (60)

LC480 Probes Master (04707494001, Roche),
**[0147]** 5 ul diluted cDNA, filled up with PCR-graded water

- PCR reactions were performed in 384 well-plates
- plates were covered by a transparent foil and spun for 3 min (3000 rpm) in a Beckman centrifuge
- plates were set into the LightCycler® 480 Instrument (Roche)
- we provided and filled in all experimental details into the appropriate program form of the LightCycler® Software Version 1.5 (Roche)

**Procedure: Relative Quantification and Data mining**

- LightCycler® Software Version 1.5 allowed the quantification of cDNA concentrations of any gene of interest in relation to internal controls (house keeping genes, like β-Actin, GAPDH and β-Globin) in treated versus non-treated (reference) samples of a certain time point based on the absolute quantification of the crossing points of their amplification curves
- Quantification was based on the formula:

$$\textbf{Normalized Ratio} = \frac{(\text{conc. gene of interest / conc. internal control})_{\text{treated sample}}}{(\text{conc. gene of interest / conc. internal control})_{\text{non-treated sample}}}$$

- values of gene expression (in arbitrary units) were transferred into Microsoft Excel Program and represented in a column diagram (gene concentration of interest in the non-treated samples at any time point set as 1 and gene concentration of interest in the treated sample set in relation to the latter)

5000 MCF-7 cells were seeded per well and 24 h later treated with a final concentration of 12.5 nM Paclitaxel (dissolved in DMSO). In comparison, control cells were treated with a final concentration of 0.1 % DMSO. At this time point cells were still in the log phase of their growth kinetics as visualized by RTCA (Figure 1), which had been validated in advance by mean of a proliferation assay using the xCELLigence® system (data not shown). The Paclitaxel concentration we applied in this experiment represents twice the IC50-value of this drug for MCF-7 cells and had been determined in an dose-response experiment (Paclitaxel titration) with the xCELLigence® system and an appropriate tool of the xCELLigence® software SP1.0.0.0807 (data not shown).
As visualized in the column diagram of Figure 2, we detected tremendous gene expression changes, specifically regulated by the impact of the tubulin-binding agent and cytostatically acting compound Paclitaxel. With respect to the first two time points (6 and 24 h), we reproduced the results of a previous study in which these genes had been found to either be up- or down-regulated in response to Paclitaxel treatment for 68% (Bani, MR, et al., Molecular Cancer Therapeutics 3(2) (2004) 111-121). We have investigated the expression of those genes at two additional time points (72 and 147 h) upon longer drug exposure. Three of the four time points are clearly preceded or paralleled by cellular changes specifically occurring in response to drug-treatment (Figure 1). The fourth time point represents the final time point of impedance-based recordings (Figure 1).
In comparison to normal situation in which cells grow from log phase, systematically reaching their plateau phase, represented by a confluent monolayer of contact-inhibited cells on the bottom of the E-plate wells, the proliferation curve of Paclitaxel-treated cells clearly drifts off from the control curve which correlates with the measurement of lower impedance or cell index values, respectively. The very immediate change in the course of the curve is based on morphological changes of the drug-treated cells. The influence of Paclitaxel on the tubulin cytoskeleton is known to lead to a rapid cell rounding and de-attachment of the cells from the culture dish which leads to a significant decrease in covered surface of the gold electrodes on the bottom of the E-plate wells. This immediate cellular effect is unlikely based on changes in gene expression of whatever sort, since the time frame would be to short for transcriptional changes of the majority of genes. And indeed, even 6h upon drug addition only small changes in expression can be determined for almost all of the investigated genes. However, 24 h after Paclitaxel treatment changes in expression levels of some of the selected genes become more obvious, as e.g. for CDKN1A, FOS, PIG8, TOP2A and MADH2 (Figure 2). This is interesting with respect to the strong change in the course of the proliferation curve at approximately 20 h upon drug addition preceding the second cell harvest. The cellular index values start to increase and the proliferation curve suddenly switches from descending to an ascending course, likely representing the phenomenon of adaptation or mitotic slippage, in which cells override the mitotic spindle checkpoint, escape the mitotic block and re-enter the G1-phase of the interphase either as aneuploid, diploid or tetraploid cells (McGrogan, B., T., et al., Biochimica et Biophysica Acta 1785 (2008) 96-132; Jordan, M., A., and Wilson, L., Curr Opin Cell Biol 10 (1998) 123-130; Dumontet, C., and Sikic, B., I., J. Clin. Oncol. 17(3)(1999) 1061-1070). In most of the currently published pharmacogenomics or - genetics studies researchers randomly focus on the detection of early drug-induced phenotypes or gene expression changes, selecting time points such as 6, 12, 24 or maximally 48 h upon drug addition (Bani, M., R., et al., Molecular Cancer Therapeutics 3(2) (2004) 111-121; Boschke, C., B., et al., Uni Tübingen (2008)). We have chosen further time points upon prolonged drug exposure, since we monitored cellular changes even around 70 h upon drug treatment. Then the proliferation curve of Paclitaxel-treated cells again changes its course, begins to descend and continues up to ca. 150 h upon drug addition. In fact we show that the strongest gene expression changes, as observed for ISG15, IFITM1, BNIP3, SLUG, ARF-1, CDC2, PLAB and IDI1, occur at these later stages of Paclitaxel treatment, which may potentially induce or at least being partially involved in the late cellular changes (Figure 1). The descending curve likely represents an increased number of de-attaching cells that may die of apoptosis during the drug-induced mitotic arrest or alternatively, because of aneuploidy as well as tetraploidy during interphase following adaptation and mitotic slippage.

Herewith, the examples shows that the combination of continuous on-line and label-free monitoring of cells through impedance-based real time cell analysis with gene expression profiling through DNA-microarray technique or q-RT-PCR allows the precise determination of the time point(s) gene expression analysis should be conducted. The observed cellular changes may not be able to be defined in their quality and extent by only real time cell analysis, but will be easier revealed by applying data sets of gene expression profiling that parallel or precede the particular cellular event together with additional methods and techniques, such as proteomics approaches or optical systems.

## Example 2

Here, a model system is described, where the online monitoring of cellular reactions after treatment with paclitaxel using the xCELLigence system is combined with qPCR analysis using the LightCycler®480 Instrument.

HT29 cells were treated either with paclitaxel or - as a control - with DMSO. The growth behavior of paclitaxel treated and control cells were monitored during the whole experiment using the xCELLigence technology. Based on the CI (cell index) profile, recorded with the xCELLigence system, time points were selected for the collection of the sample material. Subsequently, high quality RNA was purified and cDNA was synthesized. The expression level of 84 apoptosis related genes and 84 cell cycle related genes was compared for all cDNA populations with the LightCycler®480 Instrument together with the *RealTime ready Human Apoptosis Panel, 96* and the *RealTime ready Human Cell Cycle Panel, 96.*

Continuous monitoring of the growth behavior of a cell line after treatment with the anti-cancer drug paclitaxel provides a means for defining the optimal time points for the collection of sample material for subsequent analysis by RT-qPCR.

### RNA isolation from cell culture using the High Pure RNA Isolation Kit

#### Culturing the HT29 cell line and RNA Isolation

HT29 cells were cultivated in parallel in McCoy's medium in either T75 cell culture bottles (for RNA isolation) or an E-Plate 96 (for cell growth monitoring) and in three regular microtiter plates (for WST-1 assay).

The surface of the bottom of a single well of the E-Plate 96 is given with approx. 0.2 $cm^2$. T75 cell culture bottles have 75 $cm^2$. To assure comparable grow conditions within any individual well of the E-Plate 96 and the microtiter plates and within the cell culture bottles, 4.000 cells/well were seeded in the E-Plate 96 and the regular microtiter plates and 7.5 x $10^5$ cells were seeded into each T75 cell culture bottle.

| | seeding | area | Culture volume | Cell concentration |
|---|---|---|---|---|
| E-Plate 96 or regular microtiter plate | 4000 cells | 0.2 $cm^2$ | 100 μl | 40 cells/μl |
| T75 cell culture bottle | 1.5 x $10^6$ cells | 75 $cm^2$ | 37.5 ml | 40 cells/μl |

After 24 hours incubation at 37°C paclitaxel was added to a final concentration of 50 nM. As the 2 mM paclitaxel stock was dissolved in DMSO, control cells were treated with DMSO to a final concentration of 0.0025 %. In addition cells treated with medium only were monitored in parallel.

All cells were further incubated at 37°C. The growth of the cells was monitored real time on the RTCA SP Station over the whole course of the experiment (Figure 3).

Viability assay

Cells grown in the regular microtiter plates were subjected to a cell viability assay using the *Cell Proliferation Reagent WST-1.* One hour, two hours and four hours after paclitaxel treatment 10 µl WST-1 reagent were added to each well and incubated for one hour before absorption readout at 450 nm with a reference wavelength of 600 nm was carried out.

RNA Isolation and cDNA Synthesis

Cells were harvested for RNA isolation after one, two, four and 24 hours. Cell number was determined and portions of $10^6$ cells were used for RNA isolation applying the *High Pure RNA Isolation Kit* following the manufacturer's instruction.

The quality of the RNA samples was confirmed by analysis using the NanoDrop Instrument and the *Agilent Bioanalyzer.*

From each RNA population 1 µg total RNA was used for cDNA synthesis with the Transcriptor First Strand cDNA Synthesis Kit.

Real-time qPCR

The total yield of one cDNA synthesis reaction starting from 1 µg RNA was used as template for each *RealTime ready Human Apoptosis Panel, 96* or *RealTime ready Human Cell Cycle Panel, 96*. Total PCR reaction volume per well was 20 µl with Light Cycler®480 Probes Master. The easy-to-use macro for the panel containing PCR protocol, sample setup and analysis was applied on LightCycler®480 software 1.5.

Results

The cell growth of the HT29 cell line was monitored with the xCELLigence RTCA-SP system. The E-Plate 96 was loaded with 4000 cells/well in quadruplicates. As it is visible from the collected growth curve HT-29 untreated cells reach the confluent state at this cell density approx. after 70 hours (Figure 3).

To ensure that untreated cells are within the early logarithmic growth phase at the time point of paclitaxel treatment, cells were treated with 50 nM paclitaxel at approx. 1/3 of their maximum Cell Index at 20 hours after seeding.

By real time online monitoring significant changes in the Cell Index were recorded immediately after paclitaxel treatment. Interestingly the Cell Index was slightly increased within the first hour before it dropped down to reach the minimum after approx. 24 hours. Based on this data the first T75 bottle was harvested one hour after paclitaxel treatment for RNA isolation and subsequent reverse transcription and qPCR. Additional samples were collected two, four and 24 hours after paclitaxel treatment.

Cells were analyzed after one, two and four hours after paclitaxel treatment with the WST-1 assay which was carried out in parallel in regular microtiter plates. Collected data were implemented into the growth curve recorded by the xCELLigence system. (Figure 4).

This comparison clearly demonstrates the superiority of the Cell Index profile measured by the xCELLigence instrument compared to just taking three end point assays with WST-1. The WST-1 results barely reflect the quite dramatic reaction of the cells in the first hour after paclitaxel treatment. With only the WST-1 data available one would probably not have decided to isolate RNA at this early time point and missed the significant changes in RNA expression we demonstrate later.

For reliable qRT-PCR analysis high quality RNA is a crucial requirement. High quality total RNA was isolated using the *High Pure RNA Isolation Kit.* The integrity of the RNA preparation was confirmed by analysis on the *Agilent Bioanalyzer.* All samples showed high RIN values between 9.5 and 10 indicating the best prerequisite for subsequent qPCR analysis.

Four time points, one, two, four and twenty four hours were selected for qPCR runs on the LightCycler®480 system using the *RealTime Ready Human Apoptosis Panel, 96* (Figure 5) and the *RealTime Ready Human Cell Cycle Panel, 96* (Figure 7). Gene names corresponding to the numbers that can be found in the package insert of both panels (Roche Applied Science). Our data clearly demonstrate that the most significant alteration of the expression level of apoptosis related genes occurs within the first hour after paclitaxel treatment.

Comparing the data of all RealTime Ready Human Apoptosis Panel, 96 data revealed a total of 6 genes to be significantly (more than 4 times) up/down regulated (Figure 6). At two and four hours after paclitaxel treatment, no genes show significant expression changes compared to the DMSO control.

With the RealTime Ready Human Cell Cycle Panel, 96 again most dramatic effects were observed within the first four hours.

## Conclusion

The xCELLigence System records cellular events in real time without the incorporation of labels. The impedance measurement provides quantitative information about the biological status of the cells, including cell number, viability, and morphology. With xCELLigence the "body language" of the cells after a specific treatment is monitored in an online mode.

The RealTime Ready Panel is an excellent tool for extended gene expression analysis based on the Roche's Universal Probe Library. The content of each panel is especially designed for the analysis of a certain cellular pathway. A web-based tool provides background information about pathways, genes and assays to support target and assay design and contains links to public databases. Combining both new technologies provides a powerful tool for biological research.

Paclitaxel first mediates $G_2$/M-arrest and then induces apoptosis.

By monitoring the cell index changes on the xCELLigence system we were able for the first time to identify optimal time points to collect samples for subsequent gene profiling.

A typical cell viability assay like WST-1 does not reflect the significant changes in cell morphology and adhesion at early stage after drug treatment. Therefore, most probably samples for subsequent qPCR assays would not have been taken at this early time based on WST-1 data. The most important changes in gene expression would have been missed.

The evaluation of the qPCR results collected with the RealTime Ready Panels at the selected time points demonstrate, that the drop in the cell index curve is clearly correlated to significantly increased/decreased expression level of specific genes which regulate the cell cycle and initiate apoptosis.

Our results show an immediate response of the HT29 cells to the treatment with paclitaxel visualized by the changes in the cell index value.

Our data demonstrate that the combination of real time measurement of cellular growth with subsequent qRT-PCR at selected ideal time points will strongly help future research.

**Claims**

1. Method for the time resolved analysis of cells comprising

    a) providing a cell type on a sensoric surface,
    b) providing a compound,
    c) monitoring a time dependent phenotypical signature of said cells in real time using said sensoric surface after treatment of said cells with said compound,
    d) comparing in real time said time dependent phenotypical signature monitored in step c) with a predetermined phenotypical signature either obtained with the same or similar cell type or obtained with the same or similar component, said predetermined phenotypical signature comprises at least a first characteristic feature, and
    e) analyzing at least a fraction of the molecular content of said cells on said sensoric surface upon occurrence of said characteristic feature in said time dependent phenotypical signature monitored in step c).

2. The method according to claim 1, wherein upon occurrence of said characteristic feature in step e) at least a fraction of cells is removed from said sensoric surface in order to perform said analysis of the molecular content.

3. The method according to claims 1-2 comprising after step e) a further step

    f) comparing said fraction of the molecular content determined in step e) with a predetermined fraction of the same or similar molecular content obtained with either the same or similar cell type or the same or similar compound at the characteristic feature of the corresponding predetermined phenotypical signature.

4. The method according to claims 1-3, wherein said time dependent phenotypical signature is measured using an electric technique.

5. The method according to claims 1-4, wherein said predetermined phenotypical signature is obtained from a data base.

6. The method according to claims 1-5, wherein said characteristic feature of said predetermined phenotypical signature is a discontinuous change of said time dependent course or a plateau phase of said time dependent course or reaching a threshold value of said time dependent course.

7. The method according to claims 1-6, wherein said analysis in step e) is a gene expression analysis.

8. The method according to claims 1-6, wherein said analysis in step e) is a protein analysis.

9. Kit for the time resolved gene expression analysis of cells according to claims 1-7 comprising

    a) a lysis buffer, which optionally comprises a chaotropic agent,

b) reagents to perform a gene expression analysis of said cells based on PCR, and
c) a database comprising a set of predetermined phenotypical signatures together with a corresponding time dependent predetermined gene expression profile.

**10.** The kit according to claim 9, wherein said reagents to perform a gene expression analysis comprise reagents for the separation of nucleic acids from the cell debris.

**11.** System to perform the method according to claims 1-8 comprising

a) a cell analyzer for monitoring a time dependent phenotypical signature of cells in real time,
b) a database comprising a set of predetermined phenotypical signatures together with a corresponding time dependent predetermined molecular profile, said predetermined phenotypical signatures each comprises at least a first characteristic feature, and
c) a computer program to compare said time dependent phenotypical signature monitored by the cell analyzer in real time with the predetermined phenotypical signature of said database.

**12.** The system according to claim 11 further comprising an extraction device, said extraction device is arranged such that a fraction of said cells monitored in real time is extracted upon the corresponding indication from said computer program.

**13.** The system according to claims 11-12 further comprising a separation device, said separation device is arranged such that the molecular content from said cells monitored in real time is separated from the cell debris upon the corresponding indication from said computer program.

**14.** The system according to claims 11-13 further comprising an analysis device, said analysis device is arranged such that a molecular profile of said cells monitored in real time is measured upon the corresponding indication from said computer program.

**15.** The system according to claim 14, wherein said analysis device is a PCR device, preferably a real-time PCR device.

# Fig. 1

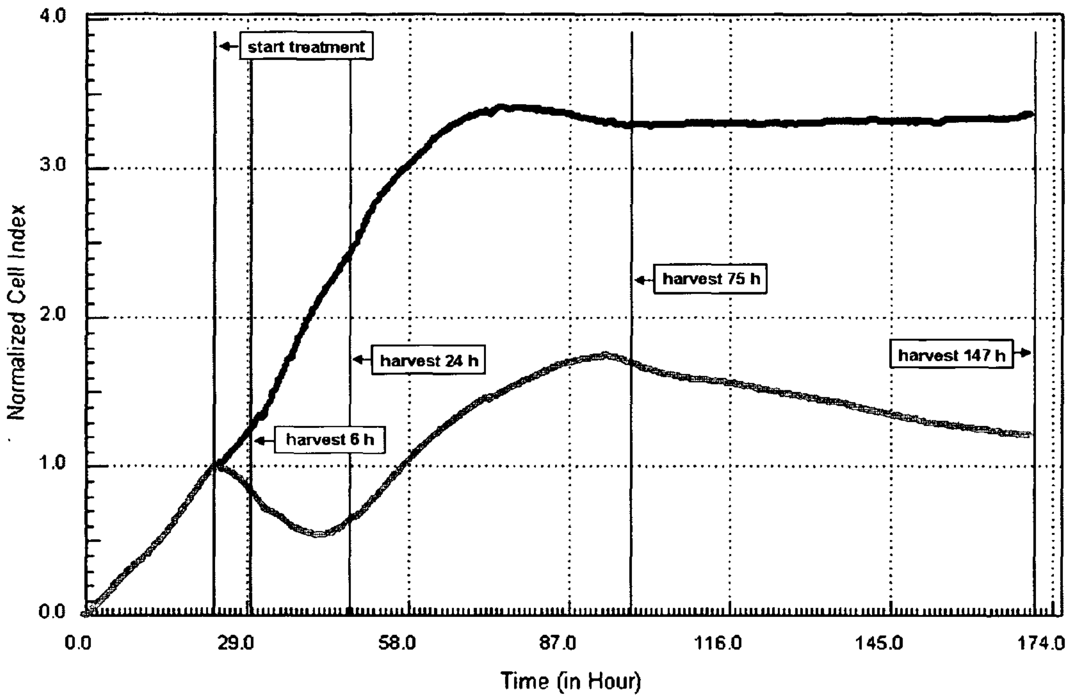
4.0
3.0
2.0
1.0
0.0
Normalized Cell Index
start treatment
harvest 75 h
harvest 24 h
harvest 147 h
harvest 6 h
0.0
29.0
58.0
87.0
116.0
145.0
174.0
Time (in Hour)

Fig. 2

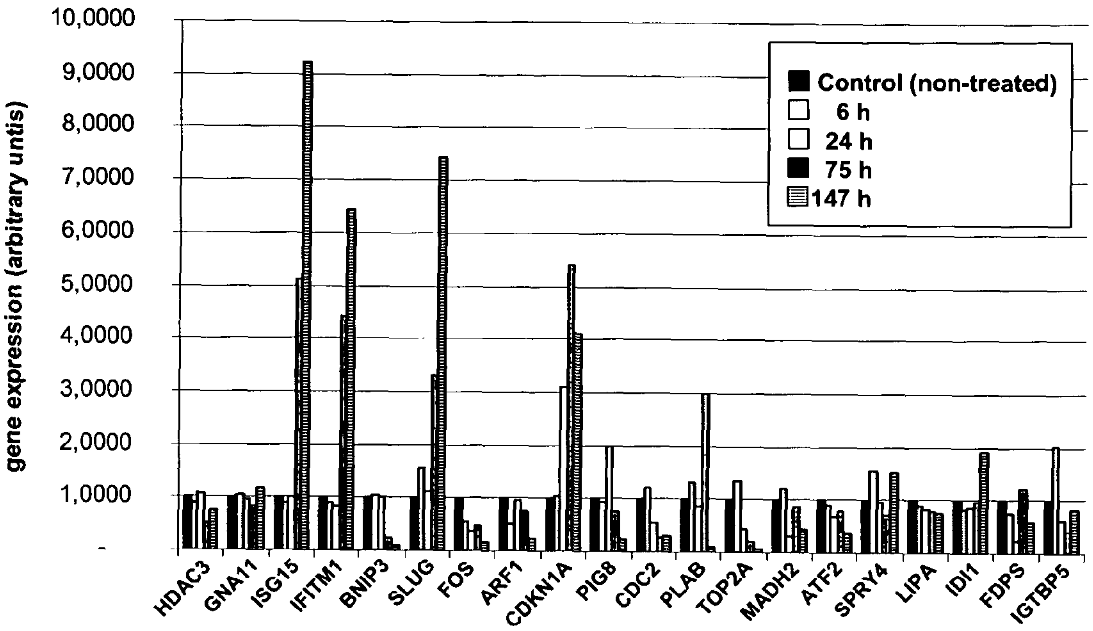
10,0000
9,0000
8,0000
7,0000
6,0000
5,0000
4,0000
3,0000
2,0000
1,0000
-
gene expression (arbitrary untis)
Control (non-treated)
6 h
24 h
75 h
147 h
HDAC3
GNA11
ISG15
IFITM1
BNIP3
SLUG
FOS
ARF1
CDKN1A
PIG8
CDC2
PLAB
TOP2A
MADH2
ATF2
SPRY4
LIPA
IDI1
FDPS
IGTBP5

Fig. 3

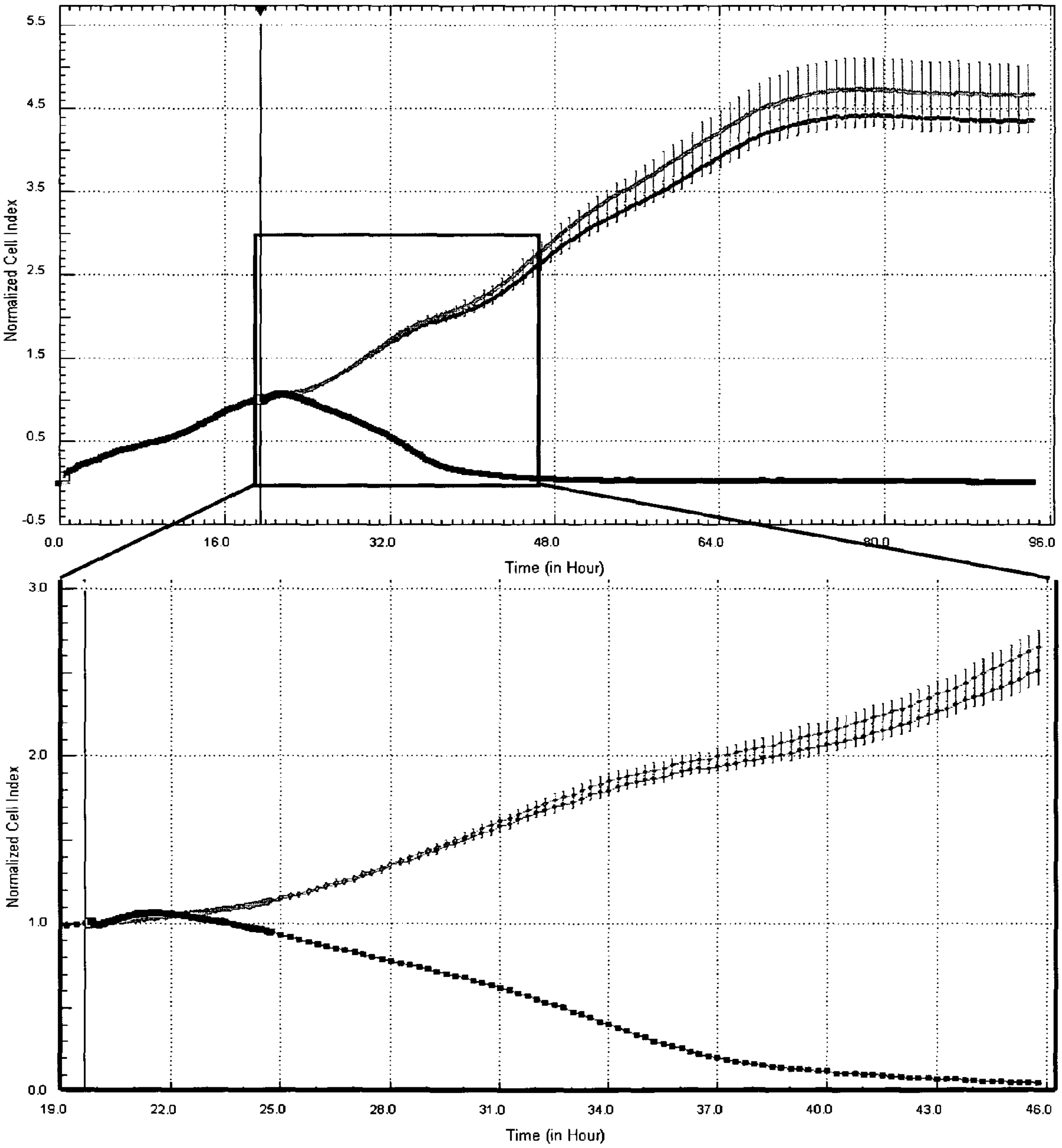
Normalized Cell Index
5.5
4.5
3.5
2.5
1.5
0.5
-0.5
0.0
16.0
32.0
48.0
64.0
80.0
96.0
Time (in Hour)
3.0
2.0
1.0
0.0
19.0
22.0
25.0
28.0
31.0
34.0
37.0
40.0
43.0
46.0

# Fig. 4

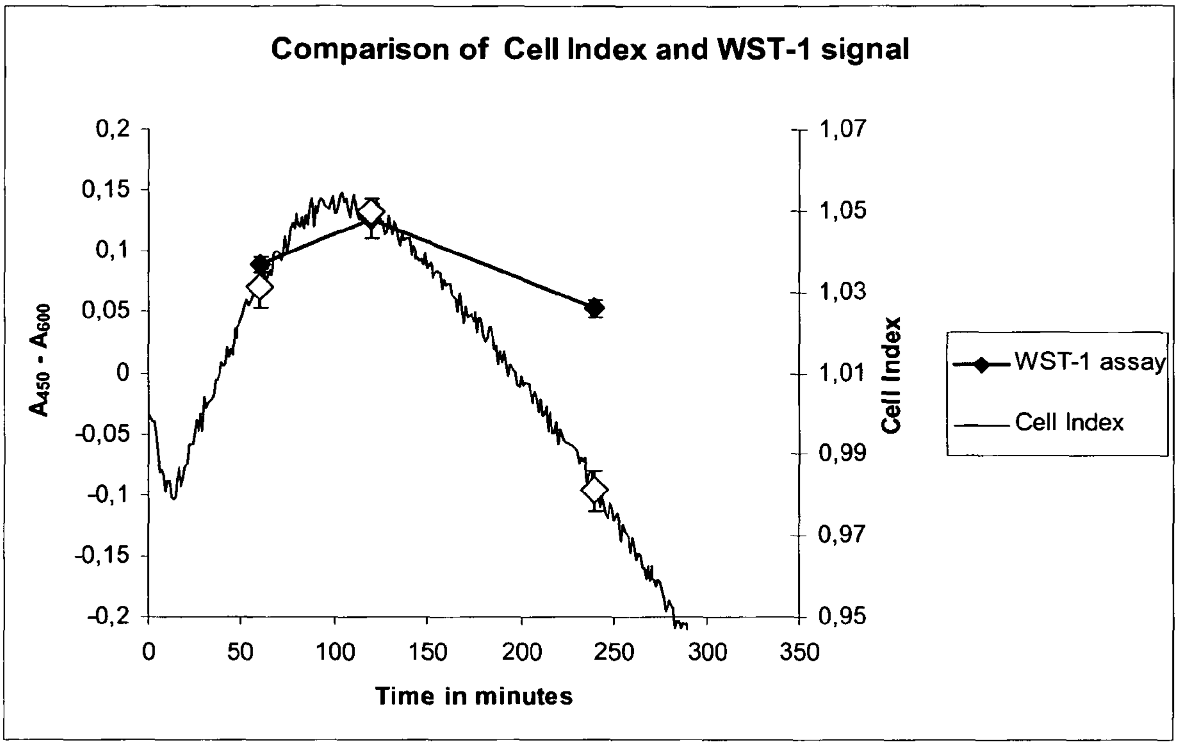
Comparison of Cell Index and WST-1 signal
0,2
0,15
0,1
0,05
0
-0,05
-0,1
-0,15
-0,2
A450 - A600
1,07
1,05
1,03
1,01
0,99
0,97
0,95
Cell Index
0
50
100
150
200
250
300
350
Time in minutes
WST-1 assay
Cell Index

Fig. 5

A)

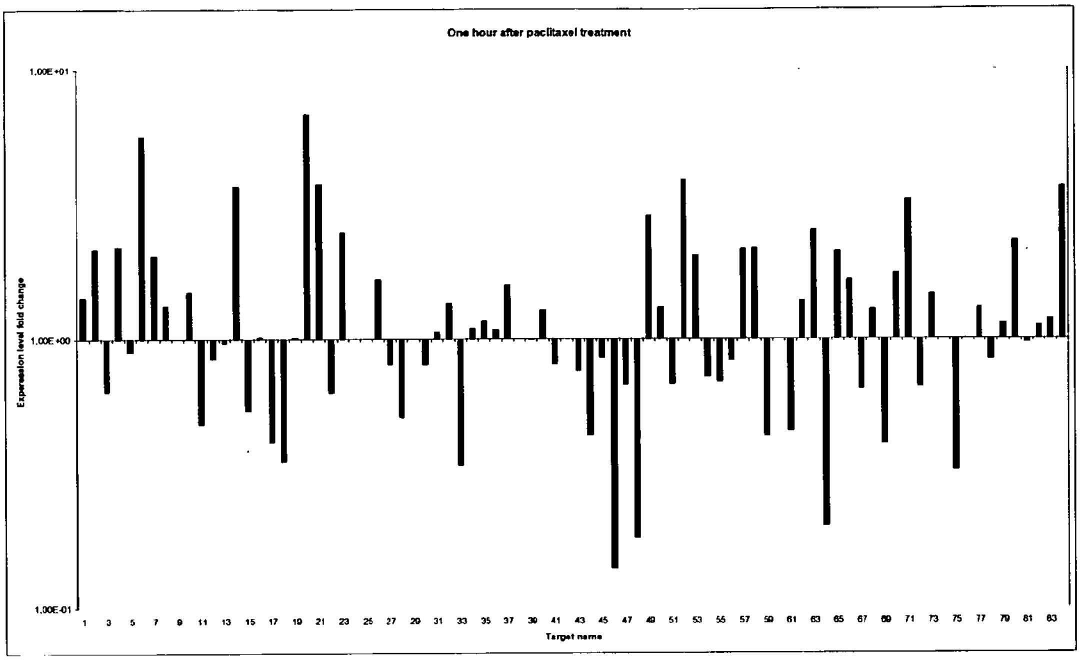
One hour after paclitaxel treatment
1,00E+01
1,00E+00
1,00E-01
Expression level fold change
Target name

B)

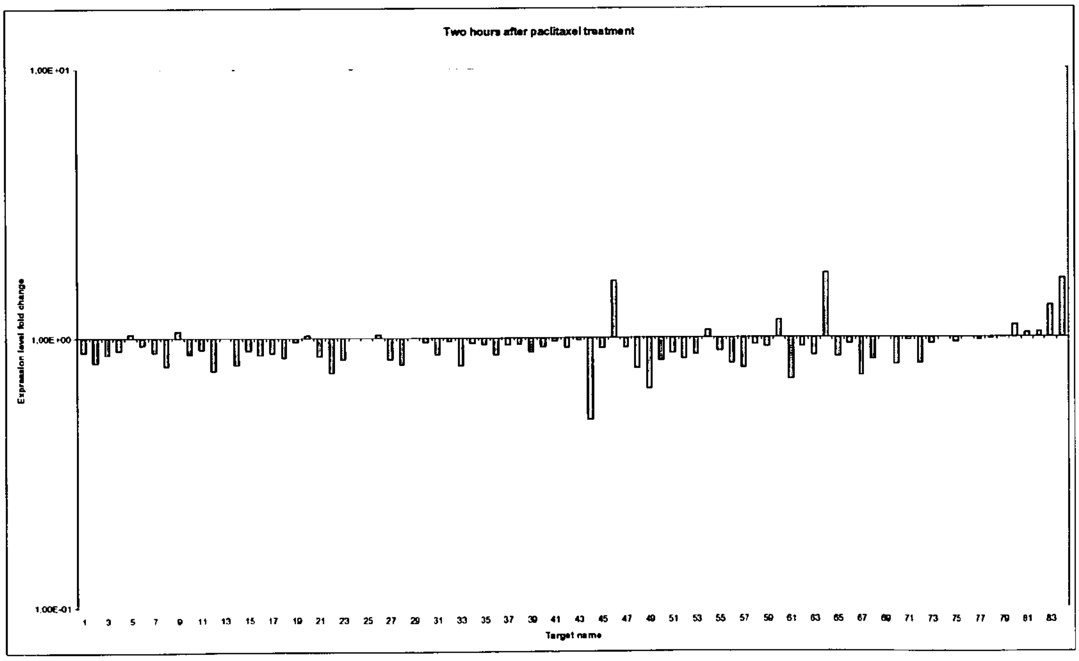
Two hours after paclitaxel treatment
1,00E+01
1,00E+00
1,00E-01
Expression level fold change
Target name

C)

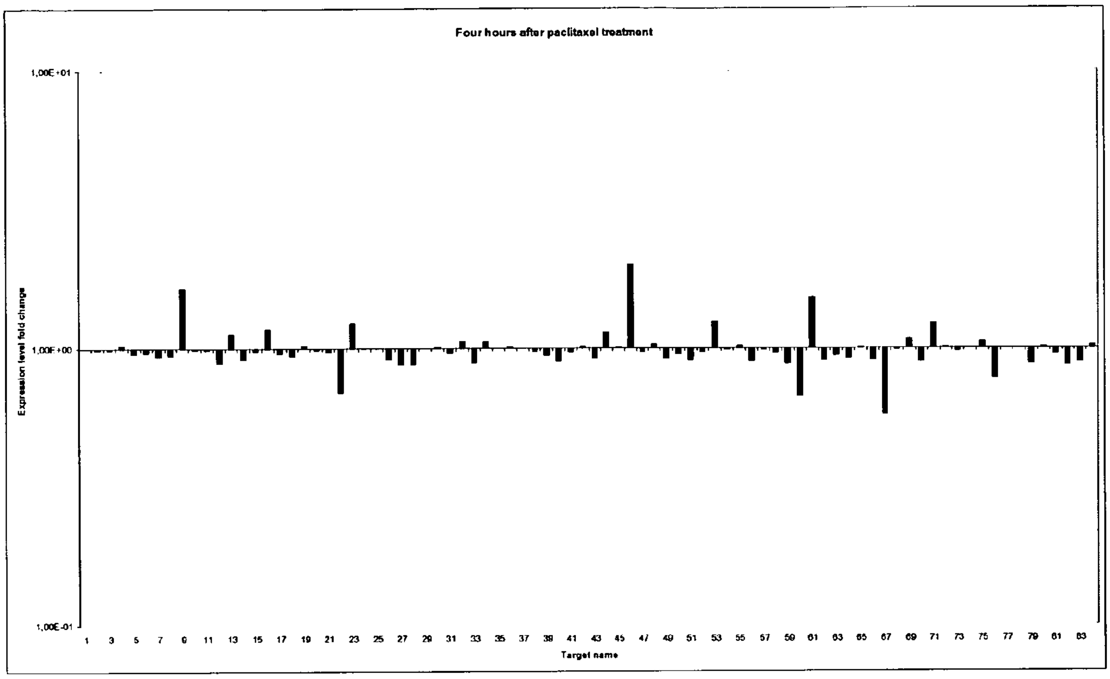
Four hours after paclitaxel treatment
1,00E+01
1,00E+00
1,00E-01
Expression level fold change
1 3 5 7 9 11 13 15 17 19 21 23 25 27 29 31 33 35 37 39 41 43 45 47 49 51 53 55 57 59 61 63 65 67 69 71 73 75 77 79 81 83
Target name

D)

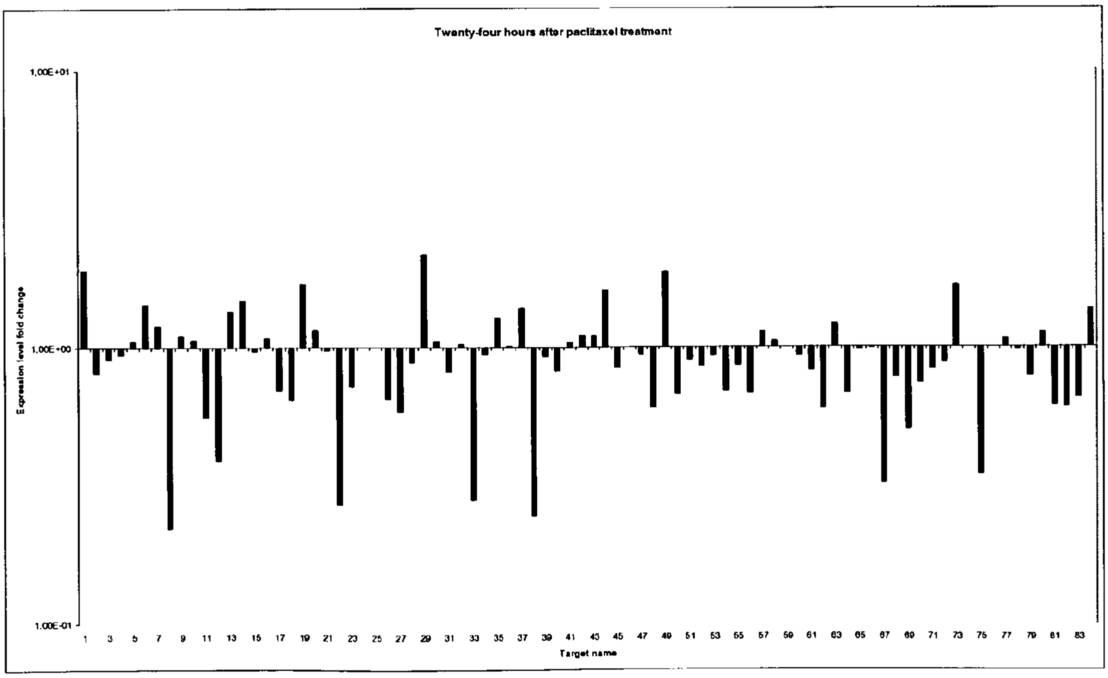
Twenty-four hours after paclitaxel treatment
1,00E+01
1,00E+00
1,00E-01
Expression level fold change
1 3 5 7 9 11 13 15 17 19 21 23 25 27 29 31 33 35 37 39 41 43 45 47 49 51 53 55 57 59 61 63 65 67 69 71 73 75 77 79 81 83
Target name

Fig. 6

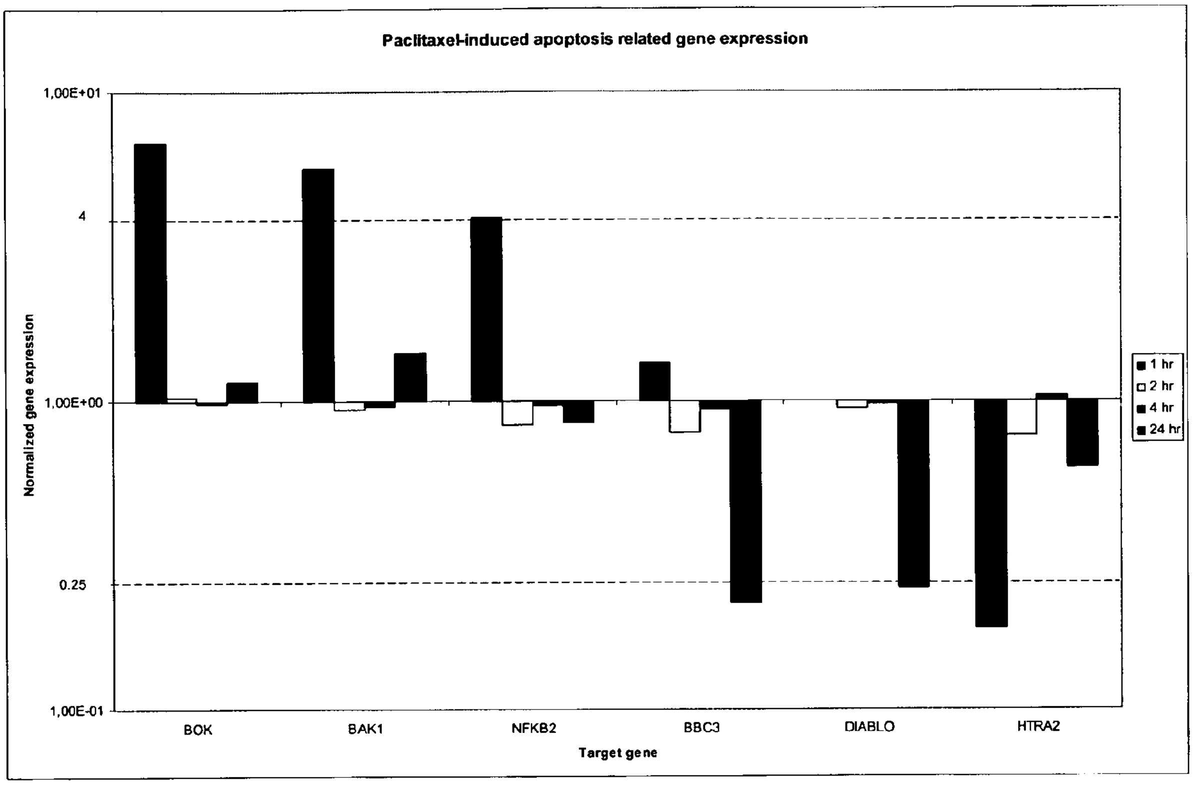
Paclitaxel-induced apoptosis related gene expression
1,00E+01
4
1,00E+00
0.25
1,00E-01
Normalized gene expression
BOK
BAK1
NFKB2
BBC3
DIABLO
HTRA2
Target gene
1 hr
2 hr
4 hr
24 hr

**Fig. 7**

A)

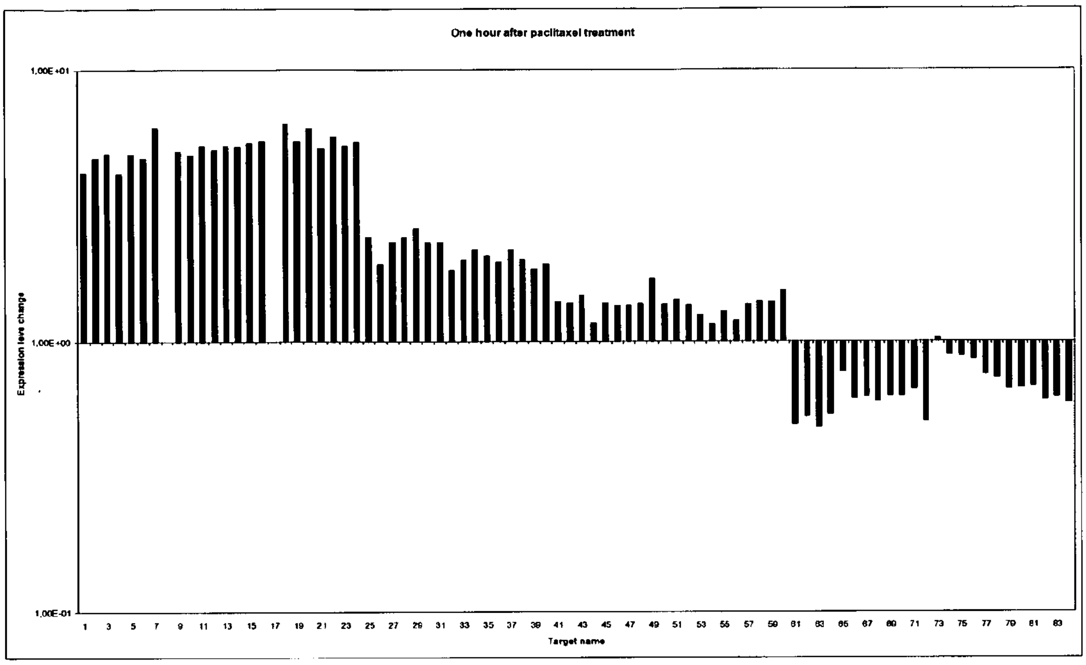
One hour after paclitaxel treatment
1,00E+01
1,00E+00
1,00E-01
Target name

B)

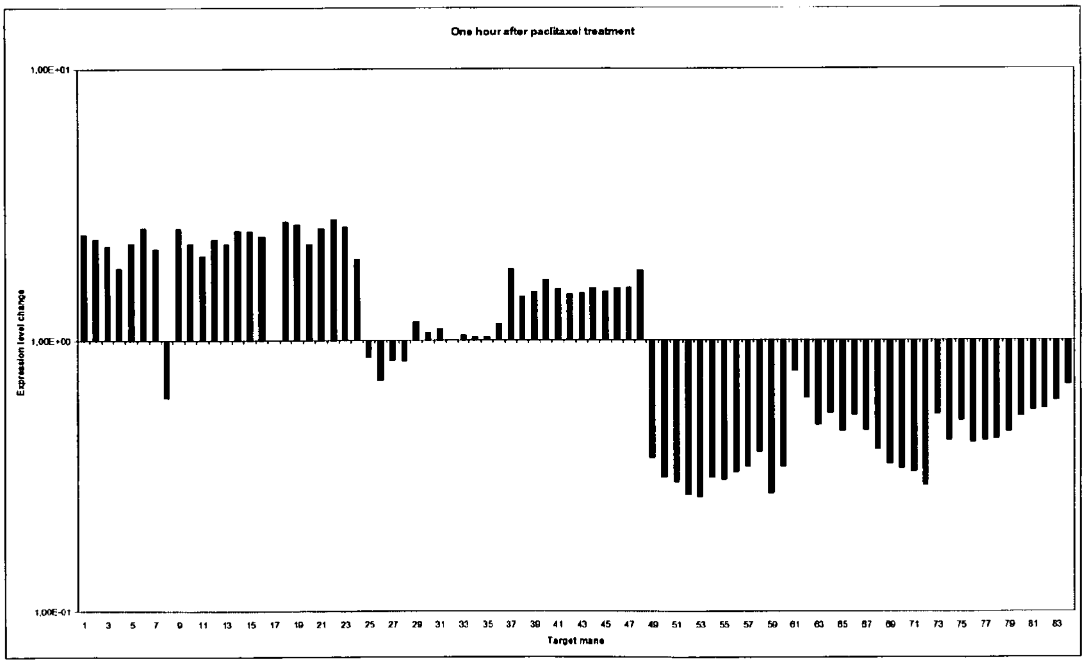
One hour after paclitaxel treatment
1,00E+01
1,00E+00
1,00E-01
Target mane

C)

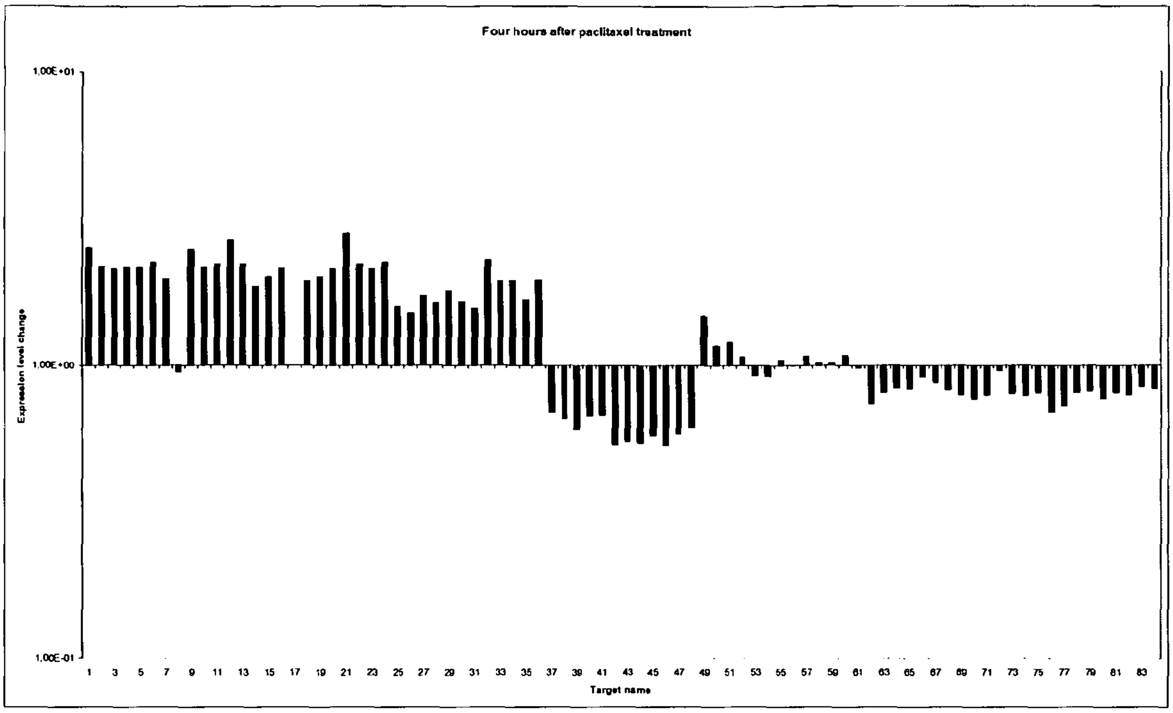
Four hours after paclitaxel treatment
1,00E+01
1,00E+00
1,00E-01
Expression level change
1 3 5 7 9 11 13 15 17 19 21 23 25 27 29 31 33 35 37 39 41 43 45 47 49 51 53 55 57 59 61 63 65 67 69 71 73 75 77 79 81 83
Target name

D)

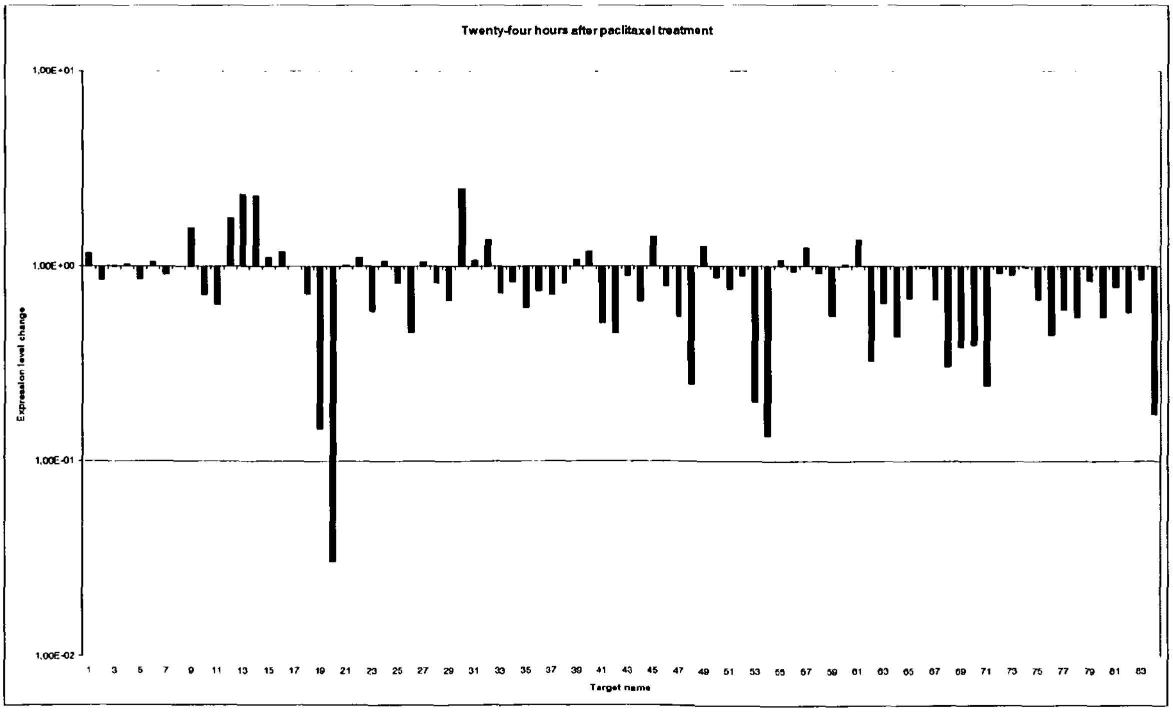
Twenty-four hours after paclitaxel treatment
1,00E+01
1,00E+00
1,00E-01
1,00E-02
Expression level change
1 3 5 7 9 11 13 15 17 19 21 23 25 27 29 31 33 35 37 39 41 43 45 47 49 51 53 55 57 59 61 63 65 67 69 71 73 75 77 79 81 83
Target name

Europäisches Patentamt
European Patent Office
Office européen des brevets

# EUROPEAN SEARCH REPORT

Application Number
EP 09 01 2973

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br>Y | ANONYMOUS: "MagNA Pure Compact System" [Online]<br>1 January 2006 (2006-01-01), , XP002513798<br>Retrieved from the Internet:<br>URL:http://roche-biochem.jp/pdf/prima/genomics/mpc.pdf><br>[retrieved on 2009-02-04]<br>* the whole document * | 9-10<br><br><br><br><br><br><br>1-8,<br>12-15 | INV.<br>G01N33/543 |
| X<br><br>Y | US 2006/023559 A1 (XU XIAO [US] ET AL)<br>2 February 2006 (2006-02-02)<br>* paragraphs [0026], [0027], [0160] * | 11<br><br>1-8,<br>12-15 | |
| X<br><br>Y | US 2005/112544 A1 (XU XIAO [US] ET AL)<br>26 May 2005 (2005-05-26)<br>* paragraphs [0181], [0236], [0246], [0247] * | 11<br><br>1-8,<br>12-15 | |
| Y | US 6 171 785 B1 (HIGUCHI RUSSELL G [US])<br>9 January 2001 (2001-01-09)<br>* the whole document * | 1-8,<br>12-15 | TECHNICAL FIELDS SEARCHED (IPC)<br>G01N |
| A | WO 2007/140015 A (ALTHEA TECHNOLOGIES INC [US]; MONFORTE JOSEPH [US])<br>6 December 2007 (2007-12-06)<br>* the whole document * | 1-15 | |

The present search report has been drawn up for all claims

| Place of search | Date of completion of the search | Examiner |
|---|---|---|
| Munich | 8 January 2010 | Dolce, Luca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 09 01 2973

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2010

| Patent document cited in search report | Publication date | Patent family member(s) | Publication date |
|---|---|---|---|
| US 2006023559 A1 | 02-02-2006 | US 2006050596 A1 | 09-03-2006 |
| US 2005112544 A1 | 26-05-2005 | US 2009205201 A1 | 20-08-2009 |
| US 6171785 B1 | 09-01-2001 | AT 184322 T | 15-09-1999 |
| | | AT 223970 T | 15-09-2002 |
| | | AU 665185 B2 | 21-12-1995 |
| | | BR 9201618 A | 15-12-1992 |
| | | CA 2067909 A1 | 03-11-1992 |
| | | CA 2218818 A1 | 03-11-1992 |
| | | DE 1256631 T1 | 27-11-2003 |
| | | DE 69229929 D1 | 14-10-1999 |
| | | DE 69229929 T2 | 18-05-2000 |
| | | DE 69232773 D1 | 17-10-2002 |
| | | DE 69232773 T2 | 07-08-2003 |
| | | DK 0512334 T3 | 03-04-2000 |
| | | DK 0872562 T3 | 30-12-2002 |
| | | EP 1256631 A1 | 13-11-2002 |
| | | EP 0512334 A2 | 11-11-1992 |
| | | EP 0872562 A1 | 21-10-1998 |
| | | ES 2137164 T3 | 16-12-1999 |
| | | ES 2183256 T3 | 16-03-2003 |
| | | JP 3136129 B2 | 19-02-2001 |
| | | JP 10201464 A | 04-08-1998 |
| | | JP 3007477 B2 | 07-02-2000 |
| | | JP 5184397 A | 27-07-1993 |
| | | NO 921731 A | 03-11-1992 |
| | | NZ 242565 A | 26-07-1994 |
| | | US 5994056 A | 30-11-1999 |
| | | US 6814934 B1 | 09-11-2004 |
| | | ZA 9202990 A | 27-01-1993 |
| WO 2007140015 A | 06-12-2007 | CA 2653321 A1 | 06-12-2007 |
| | | EP 2029781 A2 | 04-03-2009 |
| | | US 2008124726 A1 | 29-05-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0389063 A
- WO 2004010102 A
- WO 2007085353 A
- US 7192752 B
- EP 08013816 A
- US 5210015 A
- US 5538848 A
- US 5487972 A
- US 5804375 A
- US 5118801 A
- WO 9746707 A
- WO 9746712 A
- WO 9746714 A
- US 6174670 B


### Non-patent literature cited in the description


- **Sagner, G. ; Goldstein, C.** *BIOCHEMICA,* 2001, 15-17
- **Kawasaki, E.S.** *Ann. N.Y. Acad. Sci.,* 2004, vol. 1020, 92-100
- **Bengtsson, M. et al.** *Genome Research,* 2005, vol. 15, 1388-1392
- **Schlieben, S. et al.** *Bio-Nobile Oy, Technical Note Molecular Biology TN41000-003,* 2004
- **Matthews, J.A. ; Kricka, L.J.** *Analytical Biochemistry,* 1988, vol. 169, 1-25
- **Bernard, P.S. et al.** *Analytical Biochemistry,* 1998, vol. 255, 101-107
- **Burgemeister, R.** New aspects of laser microdissection in research and routine. *J Histochem Cytochem.,* 2005, vol. 53 (3), 409-12
- **Baech, J. ; Johnsen, HE.** Technical aspects and clinical impact of hematopoietic progenitor subset quantification. *Stem Cells,* 2000, vol. 18, 76-86
- **McGrogan, BT et al.** *Biochimica et Biophysica Acta,* 2008, vol. 1785, 96-132
- **Jordan, M., A ; Wilson, L.** *Curr Opin Cell Biol,* 1998, vol. 10, 123-130
- **Dumontet, C. ; Sikic, B., I.** *J. Clin. Oncol.,* 1999, vol. 17 (3), 1061-1070
- **Jordan, M., A ; Wilson, L.** *Curr. Opin. Cell. Biol.,* 1998, vol. 10, 123-130
- **Dumontet, C. ; Sikic, B., I.** *J. Clin. Oncol.,* 1999, vol. 17 (3), 1061-1070
- **McGrogan, B., T et al.** *Biochimica et Biophysica Acta,* 2008, vol. 1785, 96-132
- **Jordan, M.,A. ; Wilson, L.** *Curr Opin Cell Biol,* 1998, vol. 10, 123-130
- **Bani, MR et al.** *Molecular Cancer Therapeutics,* 2004, vol. 3 (2), 111-121
- **Bani, M., R. et al.** *Molecular Cancer Therapeutics,* 2004, vol. 3 (2), 111-121
- **Boschke, C., B. et al.** *Uni Tübingen,* 2008
- **McGrogan, B., T. et al.** *Biochimica et Biophysica Acta,* 2008, vol. 1785, 96-132
- **Jordan, M., A. ; Wilson, L.** *Curr Opin Cell Biol,* 1998, vol. 10, 123-130